(19) Europäisches Patentamt
European Patent Office
Office européen des brevets

(11) Numéro de publication: 0 133 096 B1

(12) **FASCICULE DE BREVET EUROPÉEN**

(45) Date de publication du fascicule du brevet: 04.02.87

(51) Int. Cl.⁴: C 07 K 7/00, A 61 K 37/02

(21) Numéro de dépôt: 84401477.9

(22) Date de dépôt: 12.07.84

(54) **Nouveaux dérivés de synergistines, leur préparation et les compositions pharmaceutiques qui les contiennent.**

(30) Priorité: 13.07.83 FR 8311704

(43) Date de publication de la demande: 13.02.85 Bulletin 85/7

(45) Mention de la délivrance du brevet: 04.02.87 Bulletin 87/6

(84) Etats contractants désignés: AT BE CH DE FR GB IT LI LU NL SE

(56) Documents cité:
US-A-4 355 112

PROCEEDINGS - AN INTERNATIONAL CONFERENCE ON TRENDS IN ANTIBIOTIC RESEARCH - GENETICS, BIOSYNTHESES, ACTIONS & NEW SUBSTANCES, 14-15 juin 1982, Tokyo, pages 89-98, Japan Antibiotics Research Association, Tokyo, JP; M.-L. CAPMAU et al.: "Mechanism of action of the pristinamycins"
CHEMICAL ABSTRACTS, vol. 71, no. 11, 1969, page 201, no. 47912e, Columbus, Ohio, US; M. DELEPINE: "Pristinamycin; an antibiotic with two synergystic components"
CHEMICAL ABSTRACTS, vol. 69, no. 3, 1968, page 1031, no. 10707z, Columbus, Ohio, US; J. PREUD'HOMME et al.: "Isolation, characterization, and identification of the components of pristinamycin"

(73) Titulaire: RHONE- POULENC SANTE, Les Miroirs 18 Avenue d'Alsace, F-92400 Courbevoie Cedex (FR)

(72) Inventeur: Corbet, Jean- Pierre, "Les Marronniers Résidence "Charrière Blanche", F-69140 Ecully (FR)
Inventeur: Cotrel, Claude, 17A avenue du Docteur Arnold Netter, F-75012 Paris (FR)
Inventeur: Farge, Daniel, 30 rue des Pins Sylvestres, F-94320 Thiais (FR)
Inventeur: Paris, Jean- Marc, 8 rue des Acacias, F-77360 Vaires- sur- Marne (FR)

(74) Mandataire: Gaumont, Robert, RHONE- POULENC RECHERCHES Service Brevets Pharma 25, Quai Paul Doumer, F-92408 Courbevoie Cedex (FR)

EP 0 133 096 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1987

**Description**

La pristinamycine et la virginiamycine sont des produits connus: J. Preud'homme et coll., Bull. Soc. Chim. Fr, 2, 585-91 (1968).

La présente invention concerne de nouveaux dérivés de synergistines de formule générale:

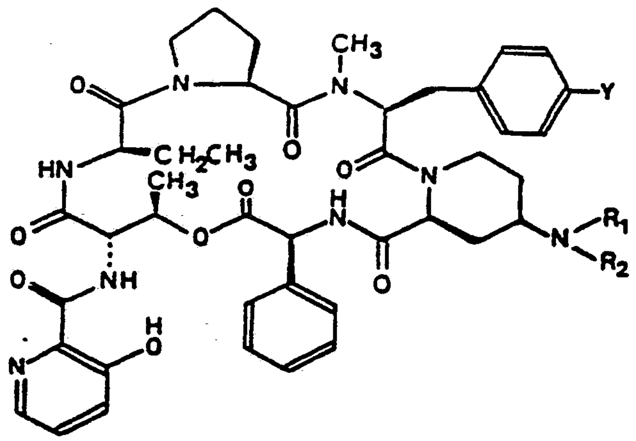

(I)

leurs sels, leur préparation et les compositions qui les contiennent.

Dans la formule générale (I), Y représente un atome d''hydrogène ou un radical diméthylamino et

- soit $R_1$ représente un atome d'hydrogène et $R_2$ représente un radical hydroxy ou alcoyle éventuellement substitué par un radical carboxy, alcoyloxycarbonyle, hydroxy, alcoylamino ou dialcoylamino dont les radicaux alcoyle peuvent former avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle, ou bien $R_2$ représente un radical cycloalcoyle contenant 3 à 7 atomes de carbone ou un hétérocycle saturé à 4 à 7 chaînons choisi parmi les cycles azétidine, pyrrolidine, pipéridine et azépine, ces hétérocycles pouvant être éventuellement substitués sur l'atome d'azote par un radical alcoyle,
- soit $R_1$ représente un radical formyle ou alcoylcarbonyle et $R_2$ représente un radical alcoyle substitué par un radical carboxy, alcoylamino ou dialcoylamino dont les radicaux alcoyle peuvent former avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle, ou bien $R_2$ représente un hétérocycle à 4 à 7 chaînons choisi parmi les cycles azétidine, pyrrolidine, pipéridine et azépine, ces hétérocycles pouvant être substitués sur l'atome d'azote par un radical alcoyle,
- soit $R_1$ et $R_2$, identiques ou différents, représentent un radical alcoyle éventuellement substitué par un radical carboxy, alcoyloxycarbonyle, hydroxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle
- soit $R_1$ et $R_2$ forment ensemble avec l'atome d'azote auquel ils sont rattachés un hétérocycle à 4 à 7 chaînons choisi parmi les cycles azétidine, pyrrolidine, pipéridine, morpholine et pipérazine éventuellement substitué par un radical alcoyle, étant entendu que tous les radicaux alcoyle et portions alcoyle cités ci-dessus ou qui seront cités par la suite sont en chaîne droite ou ramifiée et contiennent 1 à 5 atomes de carbone.

Selon l'invention, les produits de formule générale (I) à l'exception de ceux pour lesquels $R_1$ représente un radical formyle ou alcoylcarbonyle, peuvent être préparés par action d'une amine de formule générale:

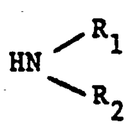

(II)

dans laquelle $R_1$ et $R_2$ sont définis comme précédemment à l'exception de représenter un radical formyle ou alcoylcarbonyle sur une symergistine de formule générale:

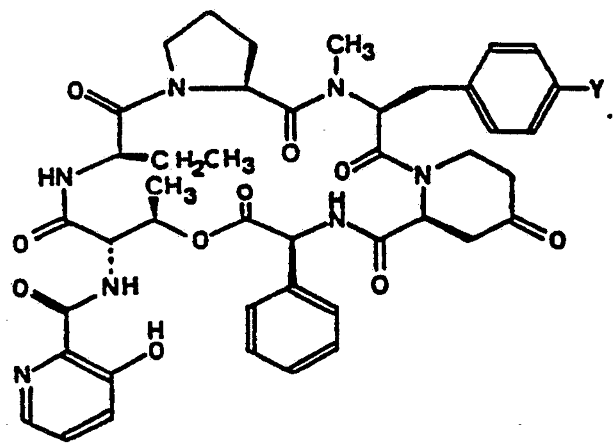

(III)

dans laquelle Y représente un atome d'hydrogène (virginiamycine S) ou le radical diméthylamino (pristinamycine $I_A$), en présence d'un cyanoborohydrure alcalin.

On opère généralement avec un excès d'amine de formule générale (II) en présence d'un cyanoborohydrure alcalin comme le ovanoborohydrure de sodium dans un solvant organique tel qu'un alcool dans lequel on a dissout de l'acide chlorhydrique gazeux (méthanol chlorhydrique ou éthanol chlorhydrique), à une température comprise entre 0°C et la température de reflux du mélange réactionnel, de préférence à une température voisine de 20°C.

La réaction peut être avantageusement effectuée en présence d'un agent de dessication tel que des tamis moléculaires.

Il est entendu que, lorsque dans la formule générale $R_1$ et/ou $R_2$ représentent un radical contenant une fonction amine secondaire, cette dernière doit être préalablement protégée avant de faire réagir le produit de formule générale (II) sur le produit de formule générale (III).

La protection et la libération de la fonction amine s'effectuent par toute méthode connue qui n'altère pas le reste de la molécule.

Il est particulièrement avantageux d'utiliser comme radical bloquant le radical trifluoracétyle; celui-ci peut ensuite être éliminé à l'aide d'une solution aqueuse de bicarbonate alcalin tel que le bicarbonate de sodium ou de potassium.

Selon l'invention, les produits de formule générale dans laquelle $R_1$ représente un radical formyle ou alcoylcarbonyle et $R_2$ représente un radical alcoyle aubstitué par un radical carboxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle ou azépinyle, ou représente un hétérocycle saturé à 4 à 7 chaînons choisi parmi les cycles azétidine, pyrrolidine, pipéridine et azépine, ces hétérocycles pouvant être substitués sur l'atome d'azote par un radical alcoyle, et Y est défini comme précédemment, peuvent être préparés par action d'un produit de formule générale:

R - CD - X (IV)

dans laquelle R représente un atome d'hydrogène ou un radical alcoyle et X représente un atome d'halogène ou un radical alcoylcarbonyloxy, sur un produit de formule générale:

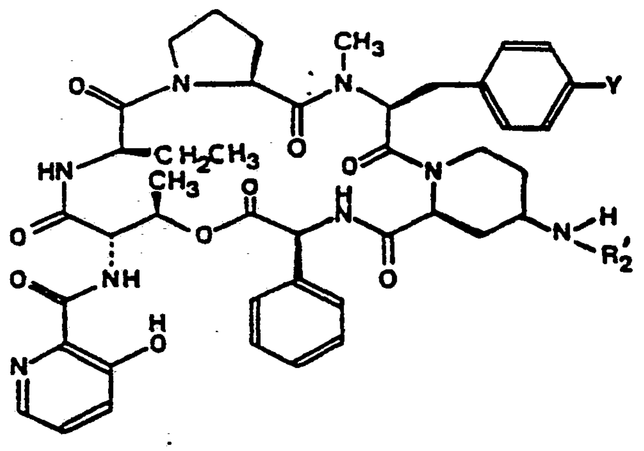

(V)

dans laquelle Y est défini comme précédemment et $R'_2$ a la définition de $R_2$ correspondante donnée ci-dessus.

La réaction s'effectue généralement dans un solvant organique tel que la pyridine, dans un solvant chloré (chlorure de méthylène) ou un éther (tétrahydrofuranne) en présence d'un accepteur d'acide tel qu'une base organique comme la triéthylamine ou une base minerale telle qu'un carbonate ou un bicarbonate alcalin comme le bicarbonate de sodium, en opérant à une température comprise entre 0° C et 80° C.

Il est entendu pour l'homme du métier que, lorsque $R'_2$ représente un radical contenant une fonction amine secondaire, ladite fonction doit être protégée avant de faire réagir le produit de formule générale (IV) sur le produit de formule générale (V). On utilise à cet effet tout moyen de blocage habituel employé pour protéger une fonction amine et pouvant être éliminé par la suite, sans toucher au reste de la molécule. On opère notamment dans les conditions décrites précédemment.

Les produits de formule générale (I) dans laquelle $R_1$ représente un atome d'hydrogène, $R_2$ représente un radical hydroxy et Y est défini comme précédemment, peuvent être également préparés par réduction des produits de formule générale:

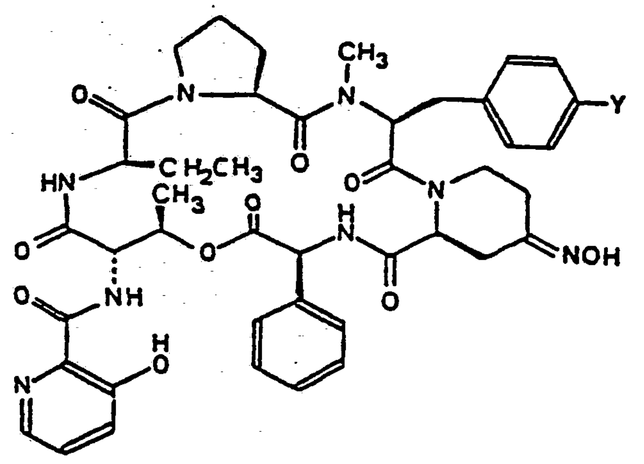

(VI)

dans laquelle Y est défini comme précédemment.

La réduction peut être effectuée par toute méthode connue pour réduire une oxime en hydroxylamine sans toucher au reste de la molécule. Il est particulièrement avantageux d'effectuer cette réduction au moyen d'un cyanoborohydrure alcalin tel que le cyanoborohydrure de sodium en solution méthanolique en présence de gaz chlorhydrique.

Les amines de formule générale (II) peuvent être obtenues par analogie avec la méthode décrite dans J. Amer. Chem. Soc., 54, 1499 (1932) et J. Amer. Chem. Soc., 54, 3441 (1932) (étant entendu que lorsque $R_1$ et/ou

$R_2$ contiennent un radical alcoylamino ce dernier est préalablement protégé par toute méthode connue qui n'altère pas le reste de la molécule) ou bien lorsque $R_1$ est un atome d'hydrogène et $R_2$ est un radical hétérocyclyle saturé à 4 à 7 chaînons, les amines de formule générale (II) peuvent être préparées par application des méthodes décrites par C.F. Elslager et coll., J. Med. Chem., 17, 99 (1974) et L.M. Werbel et coll., J. Het. Chem., 10, 363 (1973).

Les produits de formule générale (VI) peuvent être préparés par action de l'hydroxvlamine sur un produit de formule générale (III) selon les méthodes connues.

Les nouveaux produits de formule générale (I) peuvent être purifiés par les méthodes connues habituelles telles que cristallisation, chromatographie ou extractions successives en milieu acide et basique. Pour l'homme du métier connaissant la sensibilité des synergistines en milieu alcalin, il est évident qu'on entend par "milieu basique" un milieu juste suffisamment alcalin pour libérer la substance-mère de son sel d'addition avec un acide, c'est-à-dire un milieu dont le pH n'excède pas 7,5 à 8.

Les nouveaux produits de formule générale (I) dans laquelle les différents symboles sont définis comme précédemment à l'exception de ceux dans la molécule desquels $R_1$ représente un radical formyle ou alcoylcarbonyle et $R_2$ représente un radical alcoyle substitué par un radical carboxy peuvent être transformés en sels d'addition avec les acides par action d'un acide dans un solvant organique tel qu'un alcool, une cétone, un ester ou un solvant chloré. Le sel précipite, éventuellement après concentration de sa solution; il est séparé par filtration ou décantation. Les sels d'addition avec les acides peuvent également être obtenus à l'état de solutions aqueuses par addition d'une solution aqueuse d'acide correspondant sur le produit de formule générale (I) tel qu'il vient d'être défini.

Les nouveaux produits de formule générale (I) dans laquelle les radicaux $R_1$ et/ou $R_2$ représentent un radical alcoyle substitué par un radical carboxy peuvent être transformés en sels métalliques ou en sels d'addition avec les bases azotées, de manière analogue à celle décrite précédemment pour les sels d'addition avec les acides, mais en remplaçant l'acide par un hydroxyde métallique ou une base organique.

Il est bien connu que les synergistines obtenues par fermentation constituent des produits très recherchés par les médecins pour le traitement de beaucoup d'affections dues à des bactéries Gram-positives (du genre Staphylocoques, Streptocoques, pneumocoques, entérocoques) et Gram-négatives (du genre Haemophilus, gonocoques, méningocoques). Toutefois, ces produits présentent l'inconvénient d'être insolubles en milieu aqueux et ne peuvent donc être administrés que par voie orale, généralement sous forme de gellules, de dragées ou de comprimés. Compte tenu de cette insolubilité, il est impossible d'utiliser les synergistines connues lorsque le malade n'est pas en l'état de déglutir; c'est notamment le cas en pédiatrie et en réanimation, alors que le spectre d'activité de ces produits en ferait une indication précieuse dans un grand nombre de circonstances, par exemple dans les cas de septicémies comateuses.

Les nouveaux produits selon l'invention présentent l'avantage considérable de pouvoir être solubilisés dans l'eau, soit à l'état de substances-mères soit à l'état de sels, aux doses thérapeutiquement utilisables, tout en conservant le spectre général d'activité des synergistines. Ils sont notamment actifs in vitro sur Staphylococcus aureus Smith à des concentrations comprises entre 8 et 125 µg/ml.

Leur toxicité est généralement faible. Leur $DL_{50}$ est généralement supérieure à 500 mg/kg chez la souris par voie sous-cutanée.

D'un intérêt particulier sont les produits de formule générale (I) dans laquelle

Y représente un atome d'hydrogène ou un radical diméthylamino, et

- soit $R_1$ représente un atome d'hydrogène et $R_2$ représente un radical hydroxy, ou alcoyle éventuellement substitué par un radical hydroxy ou dialcoylamino ou bien $R_2$ représente un radical pipéridinyle éventuellement substitué par un radical alcoyle,
- soit $R_1$ représente un radical alcoylcarbonyle et $R_2$ représente un radical dialcoylaminoalcoyle,
- soit $R_1$ et $R_2$, identiques ou différents, représentent des radicaux alcoyle éventuellement substitués par un radical carboxy ou dialcoylamino, ou forment ensemble avec l'atome d'azote auquel ils sont rattachés, un cycle méthyl-4 pipérazinyle;

et parmi ces produits plus spécialement actifs sont les produits de formule générale (I) dans laquelle

Y représente un radical diméthylamino et

- soit $R_1$ représente un atome d'hydrogène et $R_2$ représente un radical hydroxy ou alcoyle contenant 1 ou 2 atomes de carbone ou représente un radical alcoyle pipéridinyle dont la partie alcoyle contient 1 ou 2 atomes de carbone,
- soit $R_1$ représente un radical alcoyle contenant 1 ou 2 atomes de carbone et $R_2$ représente un radical alcoyle éventuellement substitué par un radical diméthylamino,
- soit $R_1$ et $R_2$ forment ensemble avec l'atome d'azote auquel ils sont rattachés, un radical alcoylpipérazinyle dont la partie alcoyle contient 1 ou 2 atomes de carbone, et notamment les produits suivants:

- désoxy-5γ diméthylamino-5γ pristinamycine $I_A$
- désoxy-5γ méthylamino-5γ pristinamycine $I_A$
- désoxy-5γ [N-(diméthylamino-2 éthyl) N-méthylamino]-5γ pristinamycine $I_A$
- désoxy-5γ (méthyl-4 pipérazinyl-1)-5γ pristinamycine $I_A$
- désoxy-5γ hydroxyamino-5γ pristinamycine $I_A$

Pour l'emploi thérapeutique, il peut être fait usage des nouveaux produits selon l'invention tels quels, c'est-à-dire à l'état de base, mais pour l'emploi en solution aqueuse, ce qui constitue l'avantage principal des produits selon l'invention, il est particulièrement avantageux de faire usage de leurs sels pharmaceutiquement

acceptables, c'est-à-dire de sels non toxiques aux doses d'utilisation.

Comme sels pharmaceutiquement acceptables, on peut citer les sels d'addition avec les acides minéraux tels que chlorhydrates, bromhydrates, sulfates, nitrates, phosphates, ou organiques tels que acétates, propionates, succinates, maléates, fumarates, méthanesulfonates, p.toluènesulfonates, iséthionates ou des dérivés de substitution de ces composés. Comme sels pharmaceutiquement acceptables, on peut encore citer les sels avec les métaux alcalins tels que les sels de sodium, de potassium, de lithium, avec les métaux alcalinoterreux tels que le sel de magnésium, le sel d'ammonium et les sels d'addition avec les bases organiques azotées: éthanolamine, diéthanolamine, triméthylamine, triéthylamine, méthylamine, propylamine, diisopropylamine, NN-diméthyléthanolamine, benzylamine, dibenzylamine, dicyclohexylbenzylamine, N-benzyl-β-phénéthylamine, NN'-dibenzyléthylènediamine, benzhydrylamine, arqinine, leucine, lysine ou N-méthylglucamine.

Les exemples suivants, donnés à titre non limitatif, montrent comment l'invention peut être mise en pratique. Les spectres de RMN des produits illustrés dans ces exemple présentent des caractéristiques générales qui sont communes à tous les produits et des caractéristiques particulières qui sont propres à chacun des produits en fonction des substituants Y, $R_1$ et $R_2$. Dans l'exemple 1, il est donné l'attribution de tous les protons de la molécule; dans les exemples suivants ne sont mentionnées que les caractéristiques particulières dues aux radiaux variables. Tous les protons sont désignés selon la numérotation indiquée dans la formule générale (VII) et recommandée par J.O. ANTEUNIS et al [Eur. J. Biochim., 58, 259 (1975)].

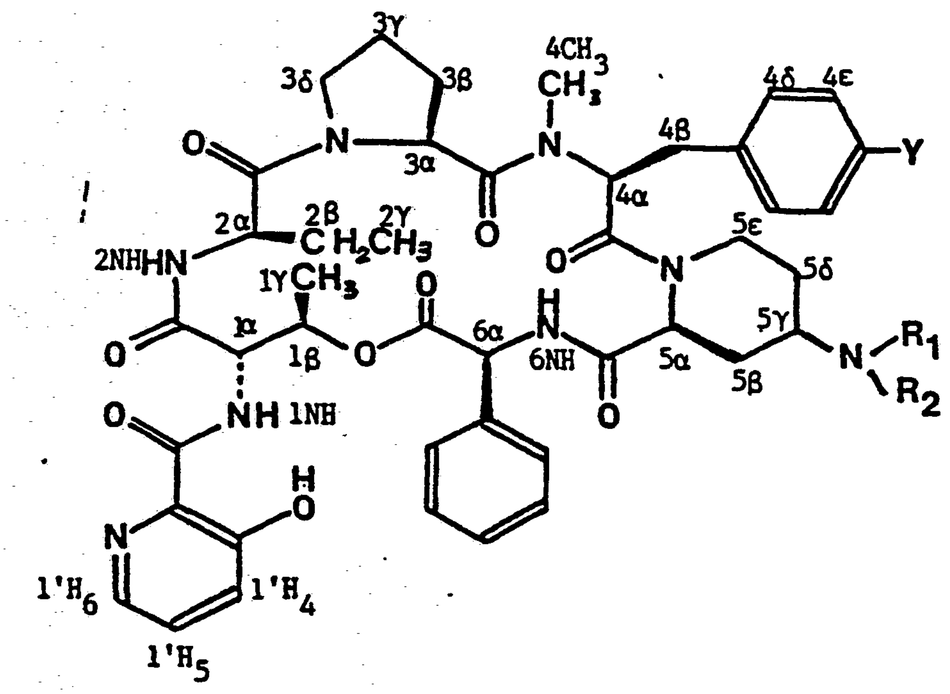

(VII)

Tous les spectres ont été faits à 250 MHz dans le deutérochloroforme; les déplacements chimiques sont exprimés en ppm par rapport au signal du tétraméthylsilane. Les abréviations utilisées dans la suite sont les suivantes:

s = singulet
d = doublet
t = triplet
mt = multiplet
m = massif
dd = doublet de doublet
dt = doublet de triplet
ddd = doublet de doublet de doublet
dddd = doublet de doublet de doublet de doublet

Dans les exemples 2 à 15 sont donnés entre parenthèses, respectivement: le déplacement chimique, la forme du signal, l'intégration (nombre de protons, avec éventuellement le pourcentage d'isomère) et l'attribution des protons.

Dans les exemples qui suivent, on appelle chromatographie "flash" une technique de purification caractérisée en ce qu'on utilise une colonne de chromatographie courte et qu'on opère sous une pression

moyenne (50 kPa) en utilisant une silice de granulométrie 40-63 µm, selon W.G.-STILL, M. KAHN et A. MITRA [J. Org. Chem., 43, 2923 (1978)].

**Exemple 1**

A une solution de 0,41 $cm^3$ de diméthylamino-3 propylamine dans 15 $cm^3$ de méthanol contenant 2,4 $cm^3$ d'une solution méthanolique 2N d'acide chlorhydrique gazeux maintenue à 55°C, on ajoute 0,5 g de pristinamycine $I_A$ et 20 mg de cyanoborohydrure de sodium. On laisse ensuite revenir la solution obtenue à une température voisine de 20°C pendant environ 2 heures, puis on la concentre à sec sous pression réduite (2,7 kPa) à 30°C. Le résidu obtenu est trituré avec un mélange de 50 $cm^3$ de chlorure de méthylène et de 50 $cm^3$ d'une solution aqueuse saturée de bicarbonate de sodium; la phase organique est décantée et la phase aqueuse est extraite 2 fois par 20 $cm^3$ au total de chlorure de méthylène. Les phases organiques sont réunies, séchées sur du sulfate de magnésium, filtrées puis concentrées à sec sous pression réduite (2,7 kPa) à 30°C. Le résidu obtenu est purifié par chromatographie "flash" [éluant: chloroforme-méthanol (80-20 en volumes)]. Les fractions 15 à 30 sont réunies et concentrées à sec sous pression réduite (2,7 kPa) à 30°C; le résidu obtenu est trituré avec 5 $cm^3$ d'éther éthylique, filtré et séché sous pression réduite (0,027 kPa) à 20°C. On obtient ainsi 60 mg de désoxy-5γ (diméthylamino-3 propyl)amino-5γ pristinamycine $I_A$ sous forme d'une poudre crème fondant vers 160°C.

Le spectre de RMN complet présente les caractéristiques suivantes:

| δ (ppm) | Forme du signal | Attribution |
|---|---|---|
| 8,40 | d | 6 NH |
| 8,25 | d | 1 NH |
| 7.55 | dd | $1'H_{\zeta}$ |
| 7,05 | m | 6γ + 6δ + 6ε |
| 7 | dd | $1'H_4$ |
| 6,90 | dd | $1'H_5$ |
| 6,70 | d | 4δ + 4ε |
| 6,40 | d | |
| 6,50 | d | 2 NH |
| 5,75 | ddd | 1β |
| 5,45 | d | 6α |
| 5,25 | dd | 4α |
| 5 | s(large) | 5α |
| 4,75 | dd | 1α |
| 4,60 | m | 2α |
| 4,45 | d(large) | $5\varepsilon_1$ |
| 4,40 | dd | 3α |
| 3,4 | dd (large) | $3\delta_1$ |
| 3,20 | dd (large) | $3\delta_2$ |
| 3 | s | 4 $CH_3$ |
| 3 | m | 5γ + $4\beta_1$ et 2 |
| 2,80 | s | 4 $N(CH_3)_2$ |
| 2,65 | t | $-NCH_2-$ (chaîne) |
| 2,35 | m | $5\varepsilon_2$ + $5\beta_1$ |
| 2,25 | t | $-NCH_2-$ (chaîne) |
| 2,20 | s | $-N(CH_3)_2$ (chaîne) |
| 1,60 | m | $-CH_2-$ (chaîne) 2β + 3γ |
| 1,25 | d | 1γ |
| 0,90 | t | 2γ |
| 0,50 | dddd | $5\beta_2$ |

On obtient une solution aqueuse à 10 % de désoxy-5γ (diméthylamino-3 propyl)amino-5γ pristinamycine $I_A$ (produit A). à l'état de chlorhydrate, avec:

produit A ........................ 0.1 g

acide chlorhydrique 2N ............. 0,52 cm3

eau distillée ...................... qsp 1 cm3

**Exemple 2**

A une solution de 2 g de pristinamycine $I_A$ dans 25 $cm^3$ de méthanol, on ajoute 2,8 $cm^3$ d'une solution éthanolique 5N de diméthylamine puis 2 $cm^3$ d'une solution méthanolique 5N d'acide chlorhydrique gazeux. A la solution ainsi obtenue, on ajoute 76 mg de cyanoborohydrure de sodium puis on agite pendant 48 heures à une température voisine de 20°C. Le mélange réactionnel est ensuite concentré à sec sous pression réduite (2,7 kPa) à 30°C. Le résidu est trituré avec un mélange de 25 $cm^3$ de chlorure de méthylène et 25 $cm^3$ d'une solution aqueuse saturée de bicarbonate de sodium; la phase organique est décantée et la phase aqueuse est extraite 2 fois par 50 $cm^3$ au total de chlorure de méthylène. Les phases organiques sont réunies, séchées sur du sulfate de magnésium, filtrées puis concentrées à sec sous pression réduite (2,7 kPa) à 30°C. Le résidu est purifié par chromatographie "flash" [élauant: chloroforme-méthanol (92-8 en volumes)]. Les fractions 5 à 12 sont réunies et concentrées à sec sous pression réduite (2,7 kPa) à 30°C. On obtient ainsi 0,7 g de désoxy-5γ diméthylamino-5γ pristinamycine $I_A$ sous forme d'une poudre beige fondant vers 170°C.

Spectre RMN :

0,70 (dt, 1H : $5\beta_2$)

2,10 à 2,60 (m, 4H : $5\delta_1$+ $5\delta_2$+ $5\beta_1$+ 5γ)

2,15 (s, 3H x 0,8 : $-N(CH_3)_2$ 1er isomère)

2,20 (s, 3H x 0,2 : $-N(CH_3)_2$ 2ème isomère)

On obtient une solution aqueuse à 2 % de désoxy-5γ diméthylamino-5γ pristinamycine $I_A$ (produit B), à l'état de chlorhydrate avec:

produit B ....................... 0.05 g

acide chlorhydrique 0,1N ......... 0,56 cm3

eau distillée .................... qsp 2,5 cm3

**Exemple 3**

En opérant d'une manière analogue à celle décrite à l'exemple 2, mais à partir de 1 g de prisitnamycine $I_A$. 1cm³ de solution éthanolique de méthylamine 7N et 0,088 g de cyanoborohydrure de sodium, et après chromatographie "flash" [éluant: chloroforme-méthanol (88-12 en volumes)] et concentration à sec des fractions 11 à 19 sous pression réduite (2,7 kPa) à 30°C, on obtient 0,35 g de désoxy-5γ méthylamino-5γ pristinamycine $I_A$ sous forme de poudre jaune fondant vers 185°C.

Spectre RMN :

0,5 (mt, 1H : $5\beta_2$)

2,4 (mt, 6H : $-NHCH_3$+ $5\delta_1$+ $5\delta_2$ + $5\beta_1$)

7,75 (mt, 1H x 0,8 : 1'$H_6$ 1er isomère)

7,97 (mt, 1H x 0,2 : 1'$H_6$ 2ème isomère)

On obtient une solution aqueuse à 1 % de désoxy-5γ méthylamino-5γ pristinamycine $I_A$ (produit C), à l'état de chlorhydrate avec:

produit C .......................... 0,05 g

acide chlorhydrique 0,1N ............ 0,57 cm3

eau distillée .................. qsp 5 cm3

**Exemple 4**

En opérant d'une manière analogue à celle décrite à l'exemple 2, mais à partir de 6 g de pristinamycine $I_A$, 5,4 $cm^3$ de (diméthylamino-2 éthyl) méthylamine, 18 $cm^3$ de solution méthanolique 5N d'acide chlorhydrique gazeux et 0,3 g de cyanoborohydrure de sodium et après purification par chromatographie "flash" [éluant: chloroforme-méthanol (85-15 en volumes)] et concentration à sec des fractions 10 à sous pression réduite (2,7 kPa) à 30°C on obtient 1,2 g de désoxy-5γ [N-(diméthylaminoéthyl)N -méthylamino]-5γ pristinamycine $I_A$ sous forme d'une poudre blanche fondant vers 120°C

Spectre RMN :

0,75 (dt, 1H : $5\beta_2$)

2,15 (s, 3H : $>N-CH_3$

2,35 (m, 7H : $-N(CH_3)_2$ + $5\beta_1$)

2,4 à 2,8 (m, 7H : $>N-CH_2-CH_2-N<$ + $5\delta_1$ + $5\delta_2$ + $5\gamma$)

7,75 (mt, 1H : 1'$H_6$ [1 seul isomère])

On obtient une solution aqueuse à 10 % de désoxy-5γ [N-(diméthylamino-2 éthyl) N-méthylamino]-5γ pristinamycine $I_A$ (produit D), à l'état de chlorhydrate, avec:

produit D .......................... 0,5 g
acide chlorhydrique 1N ............ 1,05 cm3
eau distillée ............... qsp 5 cm3

**Exemple 5**

En opérant d'une manière analogue à celle décrite à l'exemple 1, mais à partir de 10 g de pristinamycine $I_A$, 6,2 g de diméthylamino-2 éthylamine et 0,38 g de cyanoborohydrure de sodium et après purification par chromatographie "flash" [éluant: chloroforme-méthanol (88-12 en volumes)] et concentration à sec des fractions 16 à 30 sous pression réduite (2,7 kPa) à 30°C, on obtient 1,1 g de désoxy-5γ (diméthylamino-2 éthyl)amino-5γ pristinamycine $I_A$ sous forme d'une poudre jaune fondant vers 180°C.

Spectre RMN :

0,8 (m, 1H : $5\beta_2$)

2,3 (s, 6H : $-N(CH_3)_2$)

2,4 à 2,8 (m, 8H : $>N-CH_2CH_2-N<$ + $5\beta_1$ + $5\gamma$ + $5\delta_1$ + $5\delta_2$)

7,70 (mt, 1H x 0,75 : 1'$H_6$ 1er isomère)

7,95 (mt, 1H x 0,25 : 1'$H_6$ 2ème isomère)

On obtient une solution à 10 % de désoxy-5γ (diméthyl-amino-2 éthyl)amino-5γ pristinamycine $I_A$ (produit E), à l'état de chlorhydrate, avec:

produit E .......................... 0.1 g
acide chlorhydrique 0.2N ............ 0,53 cm3
eau distillée ..................qsp 1 cm3

**Exemple 6**

On ajoute 5 g de tamis moléculaire 3 A à une solution de 3 g de pristinamycine $I_A$, 3,3 g de diéthylamino-4 méthyl-1 butylamine, 0,11 g de cyanoborohydrure et 9 $cm^3$ d'une solution méthanolique 5N d'acide chlorhydrique gazeux dans 75 $cm^3$ de méthanol. La suspension obtenue est agitée pendant 4 jours à une température voisine de 20°C, puis est filtrée; le filtrat est concentré à sec sous pression réduite (2,7 kPa) à 30°C. Le résidu est trituré avec un mélange de 50 $cm^3$ de chlorure de méthylène et de 50 $cm^3$ d'une solution aqueuse saturée de bicarbonate de sodium; la phase organique est décantée et la phase aqueuse est extraite 2 fois par 50 $cm^3$ au total de chlorure de méthylène. Les phases organiques sont réunies, séchées sur du sulfate de magnésium, filtrées puis concentrées à sec sous pression réduite (2,7 kPa) à 30°C. Le résidu est purifié par chromatographie "flash" [éluant: chloroforme-méthanol (90-10 en volumes)]. On obtient ainsi 0,7 g de désoxy-5γ (diéthylamino-4 méthyl-1 butyl)amino-5γ pristinamycine $I_A$ sous forme d'une poudre beige fondant vers 160°C.

Spectre RMN :

1,10 (mt, 9H : $-N(CH_2C\underline{H}_3)_2$ + $-\underline{C}H-C\underline{H}_3$

vers 1,7 (m, 4H : $-C\underline{H}_2-C\underline{H}_2-CH_2-N(C_2H_5)_2$)

2,90 (m, 6H : $-C\underline{H}_2N(C\underline{H}_2CH_3)_2$

7,70 (mt, 1H x 0,45 : l'$H_6$ 1er isomère)

7,77 (mt, 1H x 0,55 : l'$H_6$ 2ème isomère)

On obtient une solution aqueuse à 10 % de désoxy-5γ (diéthylamino-4 méthyl-1 butyl)amino-5γ pristinamycine $I_A$ (produit F), à l'état de chlorhydrate, avec:

produit F ............................ 0,1 g

acide chlorhydrique 0,1N ......... qsp 1 cm3

**Exemple 7**

En opérant d'une manière analogue à celle décrite à l'exemple 1, mais à partir de 4 g de pristinamycine $I_A$, 2,7 g de N-méthylpipérazine et 0.16 g de cyanoborohydrure de sodium et après purification par chromatographie "flash" [éluant: chloroformeméthanol (95-5 en volumes)] et concentration à sec des fractions 12 à 23 sous pression réduite (2,7 kPa) à 30°C, on obtient 0.7 g de désoxy-5γ (méthyl-4 pipérazinyl-1)-5γ pristinamycine $I_A$ sous forme d'une poudre blanche fondant vers 195°C.

Spectre RMN :

0,8 (m, 1H : $5\beta_2$)

2,05 à 2,30 (m, 3H : $5\delta_1 + 5\delta_2$ + 5γ)

2,30 (s, 3H : $>N-C\underline{H}_3$)

2,50 (m, 9H : 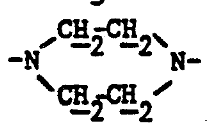 + $5\beta_1$)

7,70 (mt, 1H x 0,9 : l'$H_6$ 1er isomère)

7,98 (mt, 1H x 0,1 : l'$H_6$ 2ème isomère)

On obtient une solution aqueuse à 10 % de désoxy-5γ (méthyl-4 pipérazinyl-1)-5γ pristinamycine $I_A$ (produit G), à l'état de chlorhydrate, avec:

produit G .......................... 0,1 g

acide chlorhydrique 0,2N ............ 0,52 cm3

eau distillée ................ qsp 1 cm3

**Exemple 8**

En opérant d'une manière analogue à celle décrite à l'exemple 1, mais à partir de 6,0 g de pristinamycine $I_A$, 0,24 g de cyanoborohydrure de sodium et 4,65 g d'amino-4 méthyl-1 pipéridine et après purification par chromatographie "flash" [éluant: chloroforme-méthanol (80-20 en volumes)] et concentration à sec des fractions 12 à 36 sous pression réduite (2,7 kPa) à 30°C, on obtient 1,75 g de désoxy-5γ (méthyl-1 pipéridyl-4 amino)-5γ pristinamycine $I_A$ sous forme d'une poudre beige fondant vers 195°C.

Spectre RMN :

0.25 (ddd. 1H : 5$\beta_2$)

2,3 (m, 3H : $>$N-$CH_3$)

2,40 (d, 1H : 5ε)

3 (m, 4H : 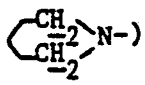)

5,10 (s, 1H : 5α)

7,75 (dd, 1H x 0,8 : 1'$H_6$ 1er isomère)

8 (dd, 1H x 0.2 : 1'$H_6$ 2ème isomère)

On obtient une solution aqueuse à 3,7 % de désoxy-5$_\gamma$ (méthyl-1 pipéridyl-4 amino)-5γ pristinamycine $I_A$ (produit H), à l'état de chlorhydrate, avec:

produit H ........................ 0,03 g

acide chlorhydrique 0,1N ......... 0,8 cm3

L'amino-4 méthyl-1 pipéridine peut être préparée selon la méthode décrite par E.F. Elslager et coll., J. Med. Chem., 17, 99(1974).

**Exemple 9**

En opérant d'une manière analogue à celle décrite à l'exemple 1, mais à partir de 2 g de pristinamycine $I_A$, 0,97 g de chlorhydrate d'hydroxylamine et 0,076 g de cyanoborohydrure de sodium et après purification par chromatographie "flash" [éluant: chloroforme-méthanol (95-5 en volumes)] et concentration à sec des fractions 10 à 17 sous pression réduite (2,7 kPa) à 30°C, on obtient 1,1 g de désoxy-5γ hydroxyamino-5γ pristinamycine $I_A$ sous forme d'une poudre blanche fondant vers 170°C.

Spectre RMN :

0,4 (m, 1H : 5$\beta_2$)

2,45 (d, 1H : 5$\beta_2$)

3,1 (d : 5γ dans un massif complexe)

7,80 (mt, 1H x 0,75 : 1'$H_6$ 1er isomère)

7,95 (mt, 1H x 0,25 : 1'$H_6$ 2ème isomère)

On obtient une solution aqueuse à 10 % de désoxy-5γ hydroxyamino-5γ pristinamycine $I_A$ (produit I), à l'état de chlorhydrate, avec:

produit I ....................... 0,1 g

acide chlorhydride 0.2N ........ 0.57 cm3

eau distillée ..............qsp 1 cm3

**Exemple 10**

A une solution de 12.5 g de désoxy-5γ hydroxyimino-5γ pristinamycine $I_A$ dans 300 cm³ de méthanol contenant 10 cm³ d'une solution méthanolique 2N d'acide chlorhydrique gazeux, on ajoute 0,7 g de cyanoborohydrure de sodium. La solution obtenue est agitée 2 jours à une température voisine de 20°C, puis concentrée à sec sous pression réduite (2,7 kPa) à 30°C. Le résidu est trituré dans un mélange de 200 cm³ de chlorure de méthylène et de 100 cm³ d'une solution aqueuse saturée de bicarbonate de sodium; la phase organique est décantée et la phase aqueuse est extraite par 100 cm³ de chlorure de méthylène. Les phases organiques sont réunies, séchées sur du sulfate de magnésium, filtrées et concentrées à sec sous pression

réduite (2,7 kPa) à 30°C. On obtient après purification par chromatographie "flash" [éluant: chloroforme-méthanol (95-5 en volumes)] 6,8 g de désoxy-5γ hydroxyamino-5γ pristinamycine $I_A$ sous forme d'une poudre blanche fondant vers 170°C.

Le spectre RMN est identique à celui obtenu à partir du produit préparé à l'exemple 9.

La désoxy-5γ hydroxyimino-5γ pristinamycine $I_A$ peut être obtenue en agitant pendant 5 heures à une température voisine de 20°C 15 g de pristinamycine $I_A$ et 7,5 g de chlorhydrate d'hydroxylamine en solution dans 150 $cm^3$ de méthanol contenant 8 $cm^3$ d'une solution méthanolique 2N d'acide chlorhydrique gazeux. Le mélange réactionnel est ensuite concentré à sec sous pression réduite (2,7 kPa) à 30°C. Le résidu est trituré avec un mélange de 100 $cm^3$ de chloroforme et de 100 $cm^3$ d'une solution aqueuse saturée de bicarbonate de sodium; la phase organique est décantée et la phase aqueuse est extraite 2 fois par 200 $cm^3$ au total de chloroforme. Les phases organiques sont réunies, séchées sur du sulfate de magnésium, filtrées et concentrées à sec sous pression réduite (2.7 kPa) à 30°C. On obtient ainsi 14 g de désoxy-5γ hydroxyimino-5γ pristinamycine $I_A$ sous forme d'une poudre beige fondant à 210°C.

Spectre RMN :

0,35 (dd, 1H : $5\beta_2$)

3,25 (m, 2H : $4\varepsilon_2 + 5\beta_1$)

5,05 (d, 1H : 5α)

5,5 (m, 2H dont $5\varepsilon_1$)

7,80 (dd, 1H x 0,40 : 1'$H_6$ 1er isomère)

7,90 (dd, 1H x 0,60 : 1'$H_6$ 2ème isomère)

**Exemple 11**

En opérant d'une manière analogue à celle décrite à l'exemple 1, mais à partir de 4 g de pristinamycine $I_A$, 3 g d'amino-3 propanol-1 et 0,16 g de cyanoborohydrure de sodium et après purification par chromatographie "flash" [éluant: chloroformeméthanol (90-10 en volumes)] et concentration à sec des fractions 9 à 16 sous pression réduite (2,7 kPa) à 30°C, on obtient 1,1 g de désoxy-5γ (hydroxy-3 propyl)amino-5γ pristinamycine $I_A$ sous forme d'une poudre crème fondant vers 160°C.

Spectre RMN :

0,45 (m, 1H : $5\beta_2$)

1,70 (m, 2H : $-CH_2-\underline{CH_2}-CH_2-$)

2,0 (m, 1H : 5δ)

2,40 (m, 2H : 5δ + $5\beta_1$)

2,90 (m, 2H :$-NH-\underline{CH_2}-$)

3,30 (m : 5γ)

3,75 (t, 2H : $-\underline{CH_2}-OH$)

7,80 (mt, 1H x 0,9 : 1'$H_6$ 1er isomère)

7,95 (mt, 1H x 0,1 : 1'$H_6$ 2ème isomère)

On obtient une solution aqueuse à 2 % de désoxy-5γ (hydroxy-3 propyl)amino-5γ pristinamycine $I_A$ (produit J), à l'état de chlorhydrate, avec:

produit J .......................... 0,1 g

acide chlorhydrique 0,1N ............. 1.08 cm3

eau distillée ...................qsp 5 cm3

**Exemple 12**

En opérant d'une manière analogue à celle décrite à l'exemple 6, mais à partir de 4 g de pristinamycine $I_A$ 2,5 g d'acide N-méthylaminoacétique et 0,076 g de cyanoborohydrure de sodium et après purification par chromatographie "flash" [éluant: chloroforme-méthanol (80-20 en volumes)] et concentration à sec des fractions 6 à 12 sous pression réduite (2,7 kPa) à 30° C, on obtient 0,8 g de [N-(carboxyméthyl)'méthylamino]-5γ désoxy-5γ pristinamycine $I_A$ sous forme de poudre crème fondant vers 140° C.

Spectre RMN :

1,15 (m, 1H : $5\beta_2$)

2,2 (m, 2H : $5\delta_1$ + $5\delta_2$)

2,40 (m, 4H : $>N-CH_3$ + $5\beta_1$)

2,8 (m : 5γ)

3,5 (m, 2H : $>N-C\underline{H}_2CO_2H$)

8,0 (mt, 1H : $1'H_6$)

On obtient une solution aqueuse à 2 % de [N-(carboxyméthyl) méthylamino]-5γ désoxy-5γ pristinamycine $I_A$ (produit K) avec:

produit K .................... 0,2 g

eau distillée ............qsp 10 cm3

**Exemple 13**

A une solution de 3,2 g de désoxy-5γ (diméthylamino-2 éthyl)amino-5γ pristinamycine $I_A$ dans 50 $cm^3$ de chloroforme contenant 0,6 $cm^3$ de triéthylamine, on ajoute 0,3 $cm^3$ de chlorure d'acétyle. Le mélange réactionnel est agité pendant 30 minutes à une température voisine de 20°C, puis concentré à sec sous pression réduite (2,7 kPa) à 30°C. Le résidu est purifié par chromatographie "flash" [éluant: chloroforme-méthanol (90-10 en volumes)]; par concentration à sec des fractions 10 à 21 sous pression réduite (2.7 kPa) à 30°C, on obtient 1,8 g de désoxy-$5_\gamma$ [N-(diméthylamino-2 éthyl)acétamido]-$5_\gamma$ pristinamycine $I_A$ sous forme d'une poudre blanche fondant vers 170° C.

Spectre RMN :

0,9 (m, 4H : 2γ + $5\beta_2$)

2,05 à 2,15 (m, 3H : $5\delta_1$+ $5\delta_2$ + 5γ)

2,15 (s, 3H : $-COCH_3$)

2,45 (s, 6H : $-N(CH_3)_2$)

2,35 à 2,60 (m, 5H : $>N-C\underline{H}_2-C\underline{H}_2-N<$ + $5\beta_1$)

7,8 (mt, 1H x 0,75 : $1'H_6$ 1er isomère)

8,25 (mt, 1H x 0,25 : $1'H_6$ 2ème isomère)

On obtient une solution aqueuse à 10 % de désoxy-5γ [N-(diméthylamino-2 éthyl) acétamido]-5γ pristinamycine $I_A$ (produit L), à l'état de chlorhydrate, avec:

produit L ......................... 0,1 g

acide chlorhydrique 0,2N ........... 0,51 cm3

eau distillée ................. qsp 1 cm3

La désoxy-5γ (diméthylamino-2 éthyl)amino-5γ pristinamycine $I_A$ peut être préparée comme décrit à l'exemple 5.

**Exemple 14**

En opérant d'une manière analogue à celle décrite à l'exemple 13, mais à partir de 2,4 g de désoxy-$5_\gamma$ (diméthylamino-3 propyl)amino-5γ pristinamycine $I_A$ et 0,2 $cm^3$ de chlorure d'acétyle et après purification par chromatographie "flash" [éluant: chloroforme-méthanol (90-10 en volumes)] et concentration à sec des fractions 13 à 18 sous pression réduite (2,7 kPa) à 30°C, on obtient 1,6 g de désoxy-5γ [N-(diméthylamino-3 propyl)acétamido] -$5_\gamma$ pristinamycine $I_A$ sous forme d'une poudre ocre fondant à 210°C.

Spectre RMN :

0,8 (m, 1H : $5\beta_2$)

1,4 (m, 2H : $-CH_2\underline{CH_2}-CH_2-$)

2,20 (s : $-COCH_3$ 1er isomère)

2,40 (s : $-COCH_3$ 2ème isomère)

2,6 (s, 6H : $-N(CH_3)_2$

2.4 à 2,6 (m. 1H : 5γ)

2,9 (m, 2H : $-\underline{CH_2}N\langle$)

7,8 (mt, 1H x 0,9 : 1'$H_6$ 1er isomère)

8,0 (mt, 1H x 0,1 : 1'$H_6$ 2ème isomère)

On obtient une solution aqueuse à 10 % de désoxy-$5_\gamma$ [N-(diméthylamino-3 propyl)acétamido]-5γ pristinamycine $I_A$ (produit M), à l'état de chlorhydrate, avec:

produit M ......................... 0,1 g

acide chlorhydrique 0,2N .......... 0,5 cm3

eau distillée ................ qsp 1 cm3

La désoxy-5γ (diméthylamino-3 propyl)amino-5γ pristinamycine $I_A$ peut être préparée comme décrit à l'exemple 1.

**Exemple 15**

En opérant d'une manière analogue à celle décrite à l'exemple 1, mais à partir de 2,5 g de virginiamycine S et 0,1 g de cyanoborohydrure de sodium et après purification par chromatographie "flash" [éluant: chloroforme-méthanol (90-10 en volumes)] et concentration à sec des fractions 8 à 15 sous pression réduite (2,7 kPa) à 30°C, on obtient 0,17 g de désoxy-5γ (diméthylamino-3 propyl) amino-5γ virginiamycine S sous forme d'une poudre beige fondant vers 140 °C.

Spectre RMN :

0,6 (ddd, 1H : $5\beta_2$)

1,65 (m, 2H : $-NHCH_2-\underline{CH_2}-CH_2N\langle$)

2,25 (s, 6H : $-CH_2-N\langle^{\underline{CH}_3}_{\underline{CH}_3}$ )

2,35 et 2,70 (m, 4H : $-NH-\underline{CH_2}-CH_2-\underline{CH_2}-N\langle$)

3,20 (d, 1H : $5\varepsilon_2$)

5,20 (m, 1H : 5α)

7,70 (dd, 1H : 1'$H_6$)

On obtient une solution aqueuse à 10 % de désoxy-5γ (diméthylamino-3 propyl)amino-5γ virginiamycine S (produit N). à l'état de chlorhydrate, avec:

produit N ........................ 10 mg
acide chlorhydrique 0,2N .......... 0,055 cm3
eau distillée ................. qsp 0,1 cm3

La présente invention concerne également les médicaments constitués par un produit de formule générale (I), sous forme libre ou sous forme de sel d'addition avec un acide ou le cas échéant une base pharmaceutiquement acceptable, à l'état pur ou sous forme d'une composition dans laquelle il est associé à tout diluant ou adjuvant pharmaceutiquement compatible. Les médicaments selon l'invention peuvent être utilisés par voie parentérale, rectale, orale ou topique.

Les compositions stériles pour administration parentérale peuvent être de préférence des solutions aqueuses ou non aqueuses, des suspensions ou des émulsions. Comme solvant ou véhicule, on peut employer l'eau, le propylèneglycol, un polyéthylèneglycol, des huiles végétales, en particulier l'huile d'olive, des esters organiques injectables, par exemple l'oléate d'éthyle ou autres solvants organiques convenables. Ces compositions peuvent également contenir des adjuvants, en particulier des agents mouillants, isotonisants, émulsifiants, dispersants et stabilisants. La stérilisation peut se faire de plusieurs façons, par exemple par filtration aseptisante, en incorporant à la composition des agents stérilisants, par irradiation ou par chauffage. Elles peuvent également être préparées sous forme de compositions solides stériles qui peuvent être dissoutes au moment de l'emploi dans un milieu stérile injectable.

Les compositions pour administration rectale sont les suppositoires ou les capsules rectales, qui contiennent outre le principe actif des excipients tels que le beurre de cacao, des glycérides semi-synthétiques ou des polyéthylèneglycols.

Comme compositions solides pour administration orale peuvent être utilisés des comprimés, des pilules, des poudres ou des granulés. Dans ces compositions, le produit actif selon l'invention (éventuellement associé à un autre produit pharmaceutiquement compatible) est mélangé à un ou plusieurs diluants ou adjuvants inertes, tels que saccharose, lactose ou amidon. Ces compositions peuvent également comprendre des substances autres que les diluants, par exemple un lubrifiant tel que le stéarate de magnésium.

Comme compositions liquides pour administration orale, on peut utiliser des émulsions pharmaceutiquement acceptables, des solutions, des suspensions, des sirops et des élixirs contenant des diluants inertes tels que l'eau ou l'huile de paraffine. Ces compositions peuvent également comprendre des substances autres que les diluants, par exemple des produits mouillants, édulcorants ou aromatisants.

Les compositions pour administration topique peuvent être par exemple des crèmes, pommades, lotions, collyres, collutoires, gouttes nasales ou aérosols.

En thérapeutique humaine, les produits selon l'invention sont particulièrement utiles dans le traitement des infections d'origine bactérienne. Les doses dépendent de l'effet recherché et de la durée du traitement. Pour un adulte elles sont généralement comprises entre 2000 et 4000 mg par jour par voie parentérale particulièrement par voie intraveineuse en perfusion lente.

D'une façon générale, le médecin déterminera la posologie qu'il estime la plus approprié en fonction de l'âge, du poids et de tous les autres facteurs propres au sujet à traiter.

Les exemples suivants, donnés à titre non limitatif, illustrent des compositions selon l'invention.

**Exemple A**

On prépare une solution injectable pour perfusion contenant 10 g/l de produit actif ayant la composition suivante:

- désoxy-5γ diméthylamino-5γ pristinamycine $I_A$ ............ 10 g
- solution aqueuse d'acide chlorhydrique 0,1N ............ 110 cm3
- eau distillée ............................ qsp 1000 cm3

**Exemple B**

On prépare une solution injectable pour perfusion contenant 5 g/l de produit actif ayant la composition suivante:

- désoxy-5γ méthylamino-5γ pristinamycine $I_A$ .............. 5 g
- solution aqueuse d'acide chlorhydrique 0,1N ............ 57 cm3
- eau distillée ............................ qsp 1000 cm3

**Revendications** pour les Etats contractants BE CH DE FR GB IT LI LU NL SE

1 - Nouveau dérivé de synergistines, caractérisé en ce qu'il répond à la formule générale:

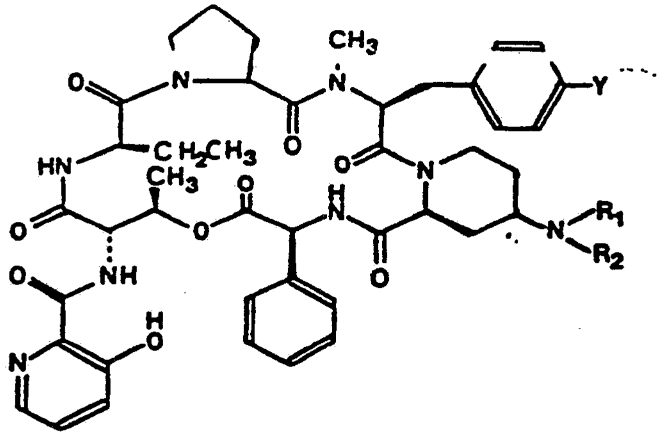

(I)

dans laquelle Y représente un atome d'hydrogène ou un radical diméthylamino et

- soit $R_1$ représente un atome d'hydrogène et $R_2$ représente un radical hydroxy ou alcoyle éventuellement substitué par un radical carboxy, alcoyloxycarbonyle, hydroxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle et azépinyle, ou bien $R_2$ représente un radical cycloalcoyle contenant 3 à 7 atomes de carbone ou un hétérocycle saturé à 4 à chaînons choisi parmi les cycles azétidine, pyrrolidine, pipéridine et azépine, ces hétérocycles pouvant éventuellement être substitués sur l'atome d'azote par un radical alcoyle,

- soit $R_1$ représente un radical formyle ou alcoylcarbonyle et $R_2$ représente un radical alcoyle substitué par un radical carboxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazi-nyle et azépinyle, ou bien $R_2$ représente un hétérocycle saturé à 4 à 7 chaînons choisi parmi les cycles azétidine, pyrrolidine, pipéridine et azépine, ces hétérocycles pouvant éventuellement être substitués sur l'atome d'azote par un radical alcoyle,

- soit $R_1$ et $R_2$, identiques ou différents, représentent un radical alcoyle éventuellement substitué par un radical carboxy, alcoyloxycarbonyle, hydroxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle azépinyle,

- soit $R_1$ et $R_2$ forment ensemble avec l'atome d'azote auquel ils sont liés un hétérocycle à 4 à 7 chaînons choisi parmi les cycles azétidine, pyrrolidine, pipéridine, morpholine et pipérazine éventuellement substitué par un radical alcoyle, tous les radicaux alcoyle et portions alcoyle contenant 1 à 5 atomes de carbone en chaîne droite ou ramifiée, ainsi que leurs sels pharmaceutiquement acceptables.

2 - Procédé de préparation de dérivés de synergistines selon la revendication 1 à l'exception de ceux pour lesquels $R_1$ représente un radical formyle ou alcoylcarbonyle, caractérisé en ce qu'on fait réagir une amine de formule générale:

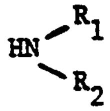

(II)

dans laquelle $R_1$ et $R_2$ sont définis comme à la revendication 1 à l'exception de représenter un radical formyle ou alcoylcarbonyle sur une synergistine de formule générale:

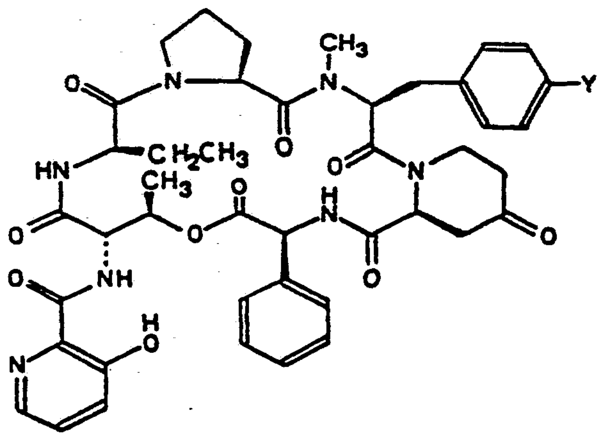

(III)

dans laquelle Y est défini comme à la revendication 1, en présence d'un cyanoborohydrure alcalin puis le cas échéant élimine le groupement protecteur d'amine, isole le produit obtenu et le transforme éventuellement en un sel pharmaceutiquement acceptable.

3 - Procédé de préparation de dérivés de synergistines selon la revendication 1 dans la formule desquels $R_1$ représente un radical formyle ou alcoylcarbonyle, $R_2$ représente un radical alcoyle substitué par un radical carboxy, alcoylamino ou dialcoylamino dont les radicaux alcoyle forment ensemble avec l'atome d'azote auquel ils sont rattachés un hétérocycle à 4 à 7 chaînons tel que défini dans la revendication 1 ou représente un hétérocycle saturé à 4 à 7 chaînons choisi parmi les cycles azétidine, pyrrolidine, pipéridine et azépine, ces hétérocycles pouvant être substitués sur l'atome d'azote par un radical alcoyle, et Y est défini comme à la revendication 1, caractérisé en ce que l'on fait réagir un produit de formule générale:

R - CO - X (IV)

dans laquelle R représente un atome d'hydrogène ou un radical alcoyle et X représente un atome d'halogène ou un radical alcoylcarbonyloxy, sur un produit de formule générale:

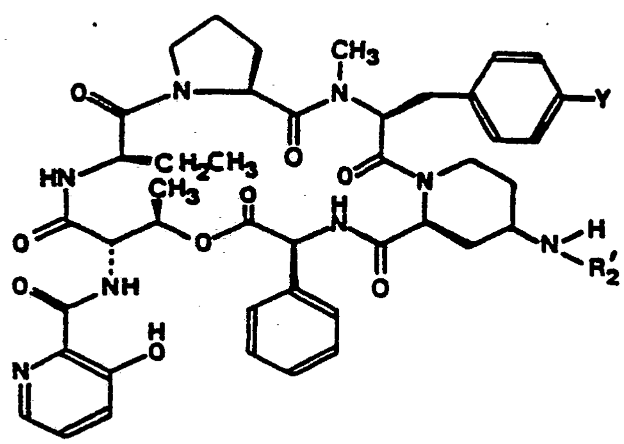

(V)

dans laquelle Y est défini comme à la revendication 1 et $R'_2$ a la définition de $R_2$ correspondante donnée ci-dessus, puis le cas échéant élimine le groupement protecteur d'amine, isole le produit obtenu et le transforme éventuellement en un sel pharmaceutiquement acceptable.

4 - Procédé de préparation de dérivés de synergistines selon la revendication 1 dans la formule desquels $R_1$ représente un atome d'hydrogène, $R_2$ représente un radical hydroxy et Y est défini comme à la revendication 1, caractérisé en ce que l'on réduit par toute méthode connue en soi un produit de formule générale:

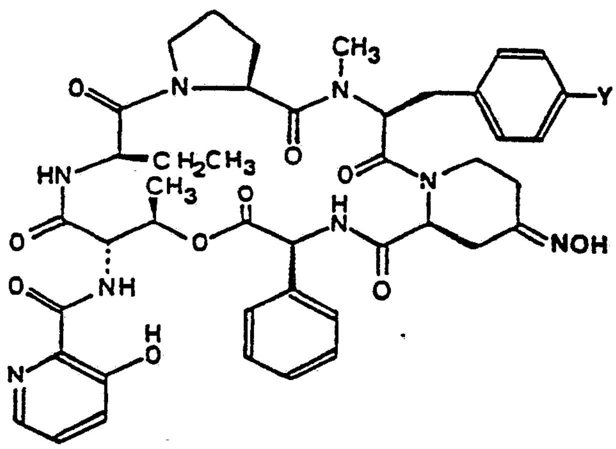

(VI)

dans laquelle Y est défini comme à la revendication 1, puis isole le produit obtenu et le transforme éventuellement en un sel pharmaceutiquement acceptable.

5 - Composition pharmaceutique caractérisée en ce qu'elle contient au moins un dérivé selon la revendication 1, en association avec un ou plusieurs diluants ou adjuvants pharmaceutiquement compatibles.

**Revendication** pour l'Etat contractant AT

Un procédé de préparation de nouveaux dérivés de synergistines de formule générale:

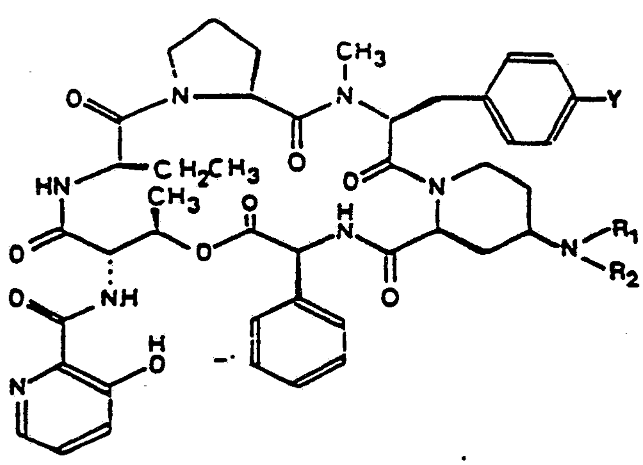

(I)

dans laquelle Y représente un atome d'hydrogène ou un radical diméthylamino et

- soit $R_1$ représente un atome d'hydrogène et $R_2$ représente un radical hydroxy ou alcoyle éventuellement substitué par un radical carboxy, alcoyloxycarbonyle, hydroxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipérioinyle, piperazinyle, N-alcoylpipérazinyle et azépinyle, ou bien $R_2$ représente un radical cycloalcoyle contenant 3 à 7 atomes de carbone ou un hétérocycle saturé à 4 à chaînons choisi parmi les cycles azétidine, pyrrolidine, pipéridine et azépine, ces hétérocycles pouvant éventuellement être substitués sur l'atome d'azote par un radical alcoyle,

- soit $R_1$ représente un radical formyle ou alcoylcarbonyle et $R_2$ représente un radical alcoyle substitué par un radical carboxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle et azépinyle, ou bien $R_2$ représente un hétérocycle saturé à 4 à 7 chaînons choisi parmi les cycles azétidine, pyrrolidine, pipéridine et azépine, ces hétérocycles pouvant éventuellement être substitués sur l'atome d'azote par un radical alcoyle,

- soit $R_1$ et $R_2$, identiques ou différents, représentent un radical alcoyle éventuellement substitué par un radical carboxy, alcoyloxycarbonyle, hydroxy, alcoylamino ou dialcoylamino dont les radicaux alcoyle forment éventuellement avec l'atome d'azote auquel ils sont rattachés un hétérocycle à 4 à 7 chaînons choisi parmi azétidinyle, pyrrolidinyle, pipéridinyle, pipérazinyle, N-alcoylpipérazinyle et azépinyle,

- soit $R_1$ et $R_2$ forment ensemble avec l'atome d'azote auquel ils sont liés un hétérocycle à 4 à 7 chaînons choisi parmi les cycles azétidine, pyrrolidine, pipéridine, morpholine et pipérazine éventuellement substitué par un radical alcoyle, tous les radicaux alcoyle et portions alcoyle contenant 1 à 5 atomes de carbone en chaîne droite ou ramifiée, ainsi que de leurs sels pharmaceutiquement acceptables,

caractérisé en ce que

lorsque $R_1$ est autre qu'un radical formyle ou alcoylcarbonyle, et $R_2$ est défini comme précédemment, on fait agir une amine de formule générale

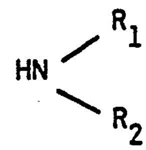

(II)

dans laquelle $R_1$ et $R_2$ sont définis comme ci-dessus sur une synergistine de formule générale:

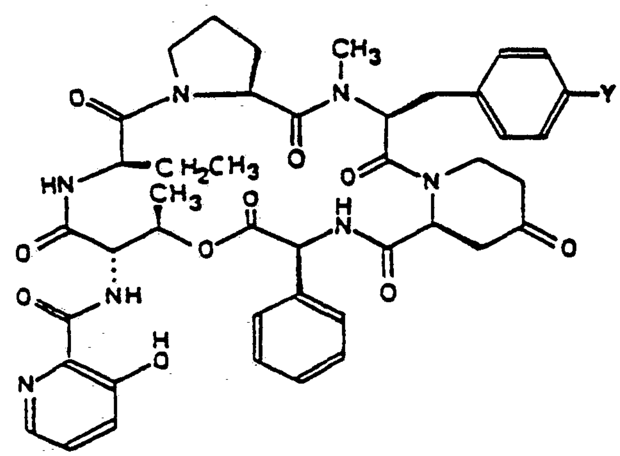

(III)

dans laquelle Y est défini comme précédemment, en présence d'un cyanoborohydrure alcalin puis le cas échéant élimine le groupement protecteur d'amine, ou bien,

lorsque $R_1$ représente un radical formyle ou alcoylcarbonyle, $R_2$ représente un radical alcoyle substitué par un radical carboxy, alcoylamino ou dialcoylamino dont les radicaux alcoyle forment ensemble avec l'atome d'azote auquel ils sont rattachés un hétérocycle à 4 à 7 chaînons tel que défini précédemment ou représente un hétérocycle saturé à 4 à 7 chaînons choisi parmi les cycles azétidine, pyrrolidine, pipéridine et azépine, ces hétérocycles pouvant être substitués sur l'atome d'azote par up radical alcoyle, et Y est défini comme précédemment, on fait agir un produit de formule générale:

R - CO - X (IV)

dans laquelle R représente un atome d'hydrogène ou un radical alcoyle et X représente un atome d'halogène ou un radical alcoylcarbonyloxy, sur un produit de formule générale:

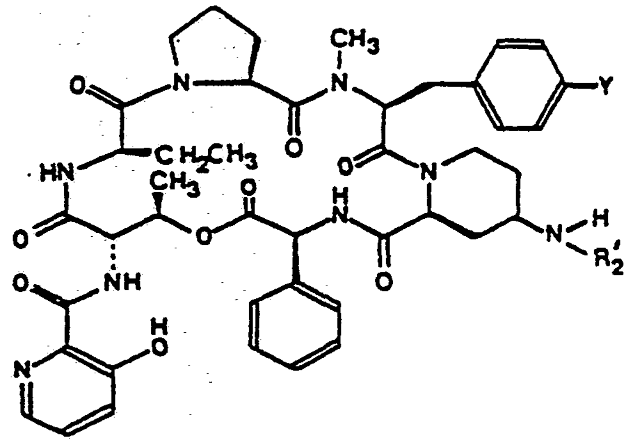

(V)

dans laquelle Y est defini comme précédemment et $R'_2$ a la définition de $R_2$ correspondante donnée ci-dessus, puis le cas échéant élimine le groupement protecteur d'amine, ou bien,

lorsque $R_1$ représente un atome d'hydrogène, $R_2$ représente un radical hydroxy et Y est défini comme précédemment, on réduit par toute méthode connue en soi un produit de formule générale:

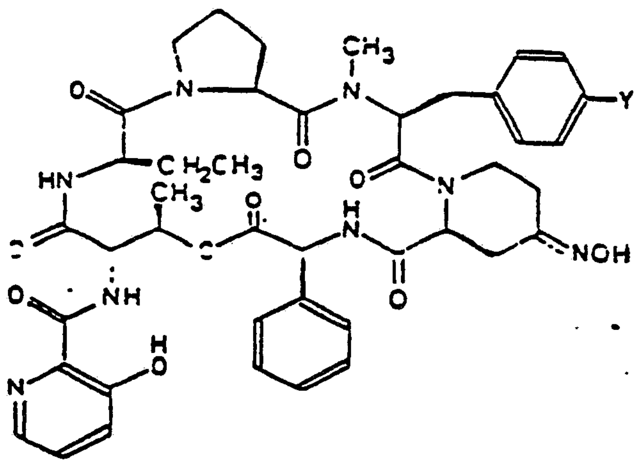

(VI)

dans laquelle Y est défini comme précédemment, puis isole le produit obtenu et le transforme éventuellement en un sel pharmaceutiquement acceptable.

**Patentansprüche** für die Vertragsstaaten

BE, CH, DE, FR, GB, IT, LI, LU, NL, SE

1. Neues Derivat von Synergistinen, dadurch gekennzeichnet, daß es der allgemeinen Formel

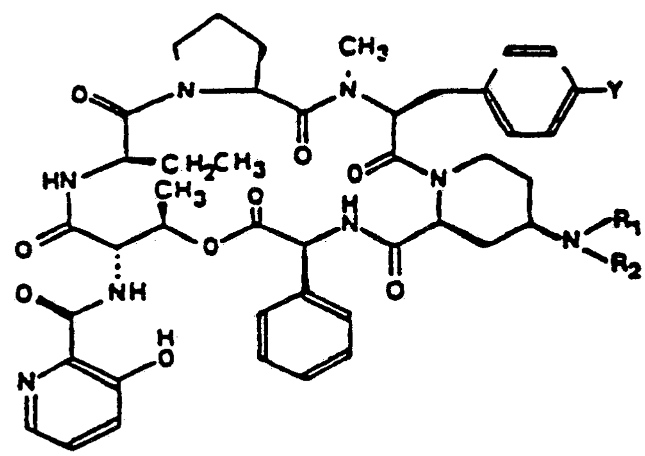

(I)

entspricht,

worin Y ein Wasserstoffatom oder einen Dimethylaminorest bedeutet, und

- entweder $R_1$ ein Wasserstoffatom bedeutet und $R_2$ bedeutet einen Hydroxy- oder Alkylrest, der gegebenenfalls durch einen Carboxy-, Alkyloxycarbonyl-, Hydroxy-, Alkylaminooder Dialkylaminorest substituiert ist, dessen Alkylreste gegebenenfalls mit dem Stickstoffatom, an das sie gebunden sind, einen Heterocyclus mit 4 bis 7 Kettengliedern bilden, ausgewählt unter Azetidinyl, Pyrrolidinyl, Piperidinyl, Piperazinyl, N-Alkylpiperazinyl und Azepinyl, oder $R_2$ bedeutet einen Cycloalkylrest mit 3 bis 7 Kohlenstoffatomen oder einen gesättigten Heterocyclus mit 4 bis 7 Kettengliedern, ausgewählt unter den Cyclen Azetidin, Pyrrolidin, Piperidin und Azepin, wobei diese Heterocyclen gegebenenfalls am Stickstoffatom durch einen Alkylrest substituiert sein können,

- oder $R_1$ bedeutet einen Formyl- oder Alkylcarbonylrest und $R_2$ bedeutet einen Alkylrest, der durch einen Carboxy-, Alkylamino- oder Dialkylaminorest substituiert ist, dessen Alkylreste gegebenenfalls mit dem

Stickstoffatom, an das sie gebunden sind, einen Heterocyclus mit 4 bis 7 Kettengliedern bilden, ausgewählt unter Azetidinyl, Pyrrolidinyl, Piperidinyl, Piperazinyl, N-Alkylpiperazinyl und Azepinyl, oder $R_2$ bedeutet einen gesättigten Heterocyclus mit 4 bis 7 Kettengliedern, ausgewählt unter den Cyclen Azetidin, Pyrrolidin, Piperidin und Azepin, wobei diese Heterocyclen gegebenenfalls am Stickstoffatom durch einen Alkylrest substituiert sein können,

- oder $R_1$ und $R_2$, die identisch oder verschieden sind, bedeuten einen Alkylrest, der gegebenenfalls durch einen Carboxy-, Alkyloxycarbonyl-, Hydroxy-, Alkylamino- oder Dialkylaminorest substituiert ist, dessen Alkylreste gegebenenfalls mit dem Stickstoffatom, an das sie gebunden sind, einen Heterocyclus mit 4 bis 7 Kettengliedern bilden, ausgewählt unter Azetidinyl, Pyrrolidinyl, Piperidinyl, Piperazinyl, N-Alkylpiperazinyl, Azepinyl,

- oder R1 und R2 bilden zusammen mit dem Stickstoffatom, an das sie gebunden sind, einen Heterocyclus mit 4 bis 7 Kettengliedern, ausgewählt unter den Cyclen Azetidin, Pyrrolidin, Piperidin, Morpholin und Piperazin, der gegebenenfalls durch einen Alkylrest substituiert ist, wobei alle Alkylreste und Alkylteile 1 bis 5 Kohlenstoffatome in gerader oder verzweigter Kette enthalten,

sowie ihre pharmazeutisch annehmbaren Salze.

. Verfahren zur Herstellung von Synergistinderivaten gemäß Anspruch 1 mit Ausnahme solcher, für die $R_1$ einen Formyl- oder Alkylcarbonylrest bedeutet, dadurch gekennzeichnet, daß man ein Amin der allgemeinen Formel

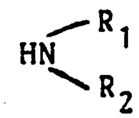

(II)

worin $R_1$ und $R_2$ wie in Anspruch 1 definiert sind, mit Ausnahme der Bedeutung eines Formyl- oder Alkylcarbonylrestes, auf ein Synergistin der allgemeinen Formel

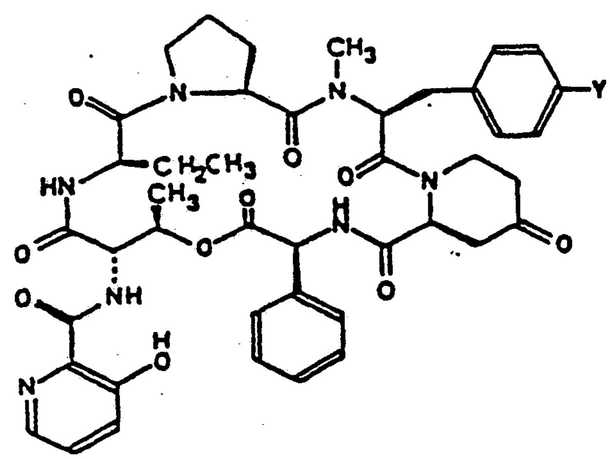

(III)

worin Y wie in Anspruch 1 definiert ist, in Gegenwart eines Alkalicyanoborhydryds einwirken läßt, und daß man dann gegebenenfalls die Aminschutzgruppe entfernt, das erhaltene produkt isoliert und es gegebenenfalls in ein pharmazeutisch annehmbares Salz überführt.

3. Verfahren zur Herstellung von Synergistinderivaten gemäß Anspruch 1, in dessen Formel $R_1$ einen Formyl- oder Alkylcarbonylrest bedeutet, $R_2$ einen Alkylrest, substituiert durch einen Carboxy-, Alkylamino- oder Dialkylaminorest, dessen Alkylreste zusammen mit dem Stickstoffatom, an das sie gebunden sind, einen Heterocyclus mit 4 bis 7 Kettengliedern bildet, wie in Anspruch 1 definiert ist, bedeutet oder einen gesättigten Heterocyclus mit 4 bis 7 Kettengliedern, ausgewählt unter den Cyclen Acetidin, Pyrrolidin, Piperidin und Azepin, bedeutet, wobei diese Heterocyclen am Stickstoffatom durch einen Alkylrest substituiert sein können, und Y wie in Anspruch 1 definiert ist, dadurch gekennzeichnet, daß man ein Produkt der Formel

R - CO - X (IV)

worin R ein Wasserstoffatom oder Alkylrest bedeutet und X ein Halogenatom oder Alkylcarbonyloxyrest bedeutet, auf ein produkt der allgemeinen Formel

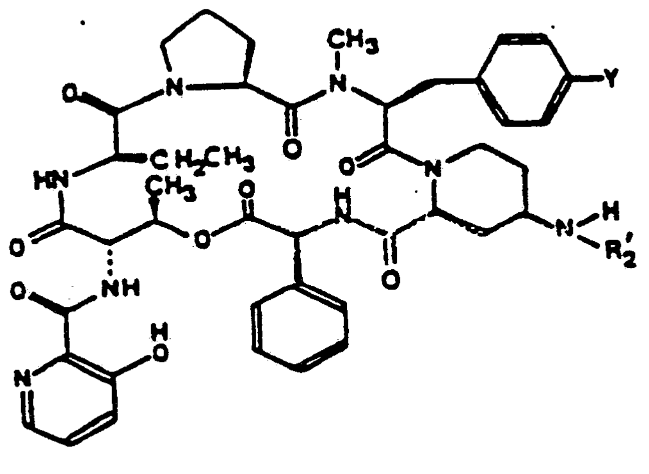

(V)

worin Y wie in Anspruch 1 definiert ist und $R'_2$ die oben angegebene entsprechende Definition von $R_2$ hat, einwirken läßt, dann gegebenenfalls die Aminschutzgruppe entfernt, das erhaltene Produkt isoliert und es gegebenenfalls in ein pharmazeutisch annehmbares Salz überführt.

4. Verfahren zur Herstellung von Synergistinderivaten gemäß Anspruch 1, in dessen Formel $R_1$ ein Wasserstoffatom bedeutet, $R_2$ einen Hydroxyrest bedeutet und Y wie in Anspruch 1 definiert ist, dadurch gekennzeichnet, daß man ein Produkt der allgemeinen Formel

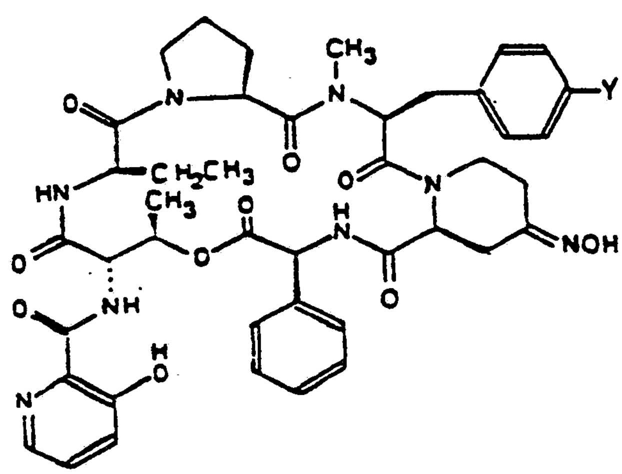

(VI)

worin Y wie in Anspruch 1 definiert ist, nach jeder an sich bekannten Methode reduziert, das erhaltene Produkt dann isoliert und es gegebenenfalls in ein pharmazeutisch annehmbares Salz überführt.

5. Pharmazeutische Zusammensetzung, dadurch gekennzeichnet, daß sie mindestens ein Derivat gemäß Anspruch 1 enthält, zusammen mit einem oder mehreren pharmazeutisch verträglichen Verdünnungs- oder Hilfsmitteln.

**Patentanspruch** für den Vertragsstaat AT

Verfahren zur Herstellung neuer synergistischer Derivate allgemeinen Formel:

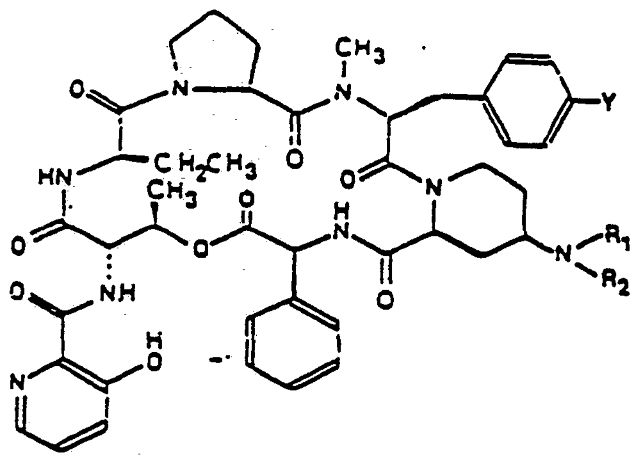

(I),

in welcher Y ein Wasserstoffatom oder eine Dimethylaminogruppe bedeutet und

- entweder $R_1$ bedeutet ein Wasserstoffatom und $R_2$ bedeutet eine Hydroxygruppe oder eine Alkylgruppe, gegebenenfalls substituiert eine Carboxy-, Alkyloxycarbonyl-, Hydroxy-, Alkylaminooder Dialkylaminogruppe, deren Alkylgruppen mit dem Stickstoffatom, an das sie gebunden sind, gegebenenfalls einen 4 bis 7 giedrigen Heterocyclus, ausgewählt unter Azetidinyl, Pyrrolidinyl, Piperidinyl, Piperazinyl, N-Alkylpiperazinyl und Azepinyl, bilden oder $R_2$ bedeutet eine Cycloalkylgruppe mit 3 bis 7 Kohlenstoffatomen oder einen gesättigten 4 bis 7 gliedrigen Heterocyclus, ausgewählt unter den Azetidin-, Pyrrolidin-, Piperidin- und Azepinringen, wobei diese Heterocyclen gegebenenfalls am Stickstoffatom durch eine Alkylgruppe substituiert sein können, - oder $R_1$ bedeutet eine Formyl- oder Alkylcarbonylgruppe und $R_2$ bedeutet eine Alkylgruppe, substituiert durch eine Carboxy-, Alkylamino- oder Dialkylaminogruppe, deren Alkylgruppen gegebenenfalls mit dem Stickstoffatom, an das sie gebunden sind, einen 4 bis 7 gliedrigen Heterocyclus, ausgewählt unter Azetidinyl, Pyrrolidinyl, Piperidinyl, Piperazinyl, N-Alkylpiperazinyl und Azepinyl, bilden, oder $R_2$ bedeutet einen gesättigten 4 bis 7 gliedrigen Heterocyclus, ausgewählt unter den Azetidin-,Pyrrolidin-, Piperidin-und Azepinringen, wobei diese Heterocyclen gegebenenfalls am Stickstoffatom durch eine Alkylgruppe substituiert sein können,

- oder $R_1$ und $R_2$, die gleich oder verschieden sind, bedeuten eine Alkylgruppe, gegebenenfalls substituiert durch eine Carboxy-, Alkyloxycarbonyl-, Hydroxy-, Alkylamino- oder Dialkylaminogruppe, deren Alkylgruppen gegebenenfalls mit dem Stickstoffatom, das das sie gebunden sind, einen 4 bis 7 gliedrigen Heterocyclus, ausgewählt unter Azetidinyl, Pyrrolidinyl, Piperidinyl, Piperazinyl, N-Alkylpiperazinyl und Azepinyl, bilden,

- oder $R_1$ und $R_2$ bilden zusammen mit dem Stickstoffatom, an das sie gebunden sind, einen 4 bis 7 gliedrigen Heterocyclus, ausgewählt unter den Azetidin, Pyrrolidin; Piperidin; Morpholin- und Piperazinringen, gegebenenfalls substituiert durch eine Alkylgruppe, wobei alle Alkylgruppen und Alkylteile 1 bis 5 Kohlenstoffatome in gerader oder verzweigter Kette enthalten, sowie ihrer pharmazeutisch zulässigen Salze, dadurch gekennzeichnet, daß man

wenn $R_1$ verschieden von einer Formyl- oder Alkylcarbonylgruppe ist und $R_2$ wie oben definiert ist, ein Amin der allgemeinen Formel:

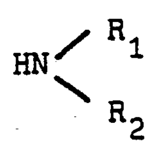

(II),

in welcher $R_1$ und $R_2$ wie oben definiert sind, mit einer synergistischen Verbindung der allgemeinen Formel:

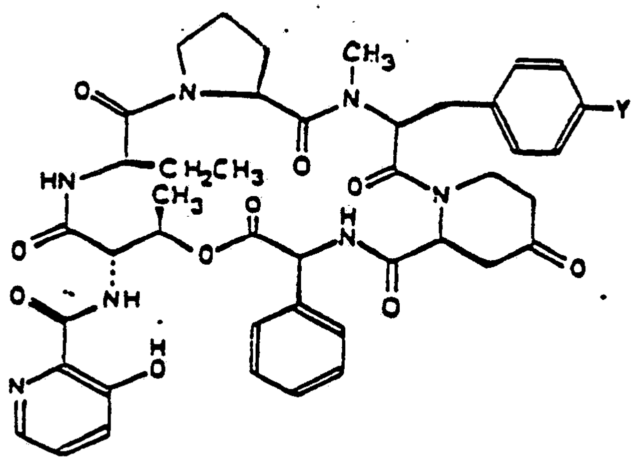

(III),

in welcher Y wie oben definiert ist, in Gegenwart eines Alkalicyanoborohydrids umsetzt und dann zutreffendenfalls die Schutzgruppe des Amins entfernt, oder daß man,

wenn $R_1$ eine Formyl- oder Alkylcarbonylgruppe bedeutet, $R_2$ eine Alkylgruppe, substituiert durch eine Carboxy-, Alkylaminooder Dialkylaminogruppe, deren Alkylreste zusammen mit dem Stickstoffatom, an das sie gebunden sind, einen 4 bis 7 gliedrigen Heterocyclus, wie sie oben definiert sind, bedeutet oder einen gesättigten 4 bis 7 gliedrigen Heterocyclus, ausgewählt unter den Azetidin-, Pyrrolidin-, Piperidin- und Azepinringen, wobei diese Heterocyclen am Stickstoffatom durch eine Alkylgruppe substituiert sein können, bedeutet

und Y wie oben definiert ist, eine Verbindung der allgemeinen Formel:

R - CO - X (IV),

in welcher R ein Wasserstoffatom oder eine Alkylgruppe bedeutet und X ein Halogenatom oder eine Alkylcarbonyloxygruppe bedeutet, mit einer Verbindung der allgemeinen Formel:

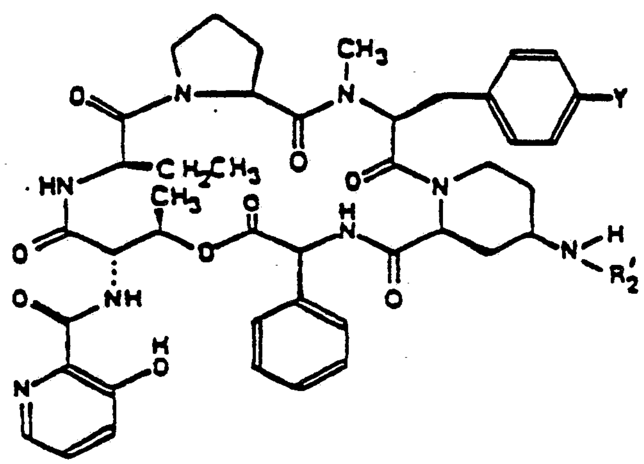

(V),

in welcher Y wie oben definiert ist und $R'_2$ die oben angegebene entsprechende Definition von $R_2$ hat, umsetzt und dann zutreffendenfalls die Schutzgruppe des Amins entfernt, oder daß man, wenn $R_1$ ein Wasserstoffatom bedeutet, $R_2$ eine Hydroxygruppe bedeutet und Y wie oben definiert ist, nach jeder an sich bekannten Methode eine Verbindung der allgemeinen Formel:

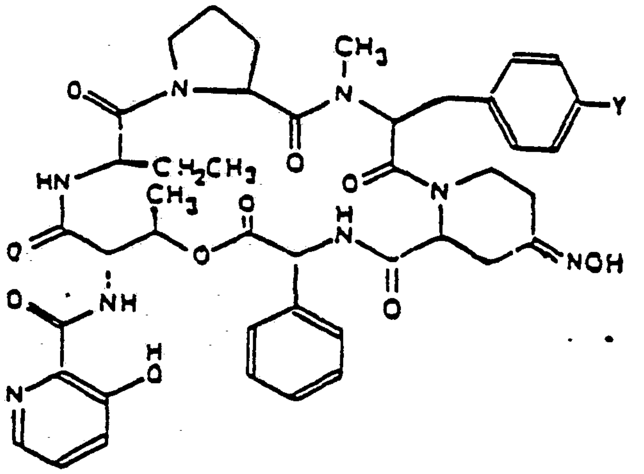

(VI)

in welcher Y wie vorstehend definiert ist, reduziert und dann die erhaltene Verbindung isoliert und sie gegebenenfalls in ein pharmazeutisch zulässiges Salz überführt.

**Claims for the contracting states BE CH DE FR GB IT LI LU NL SE**

1. New synergistin derivative, characterized in that it corresponds to the general formula:

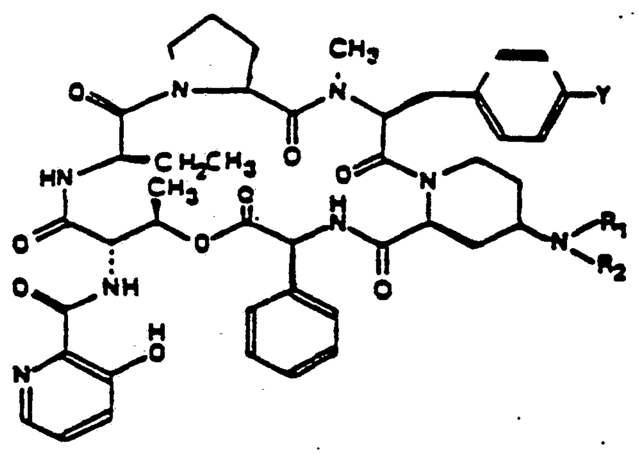

(I)

in which Y denotes a hydrogen atom or a dimethylamino radical and

either $R_1$ denotes a hydrogen atom and $R_2$ denotes a hydroxy radical or alkyl radical optionally substituted with a carboxy, alkyloxycarbonyl, hydroxy, alkylamino or dialkylamino radical in which the alkyl radicals optionally form, with the nitrogen atom to which they are attached, a 4-to 7-membered heterocyclic radical chosen from azetidinyl, pyrrolidinyl, piperidyl, piperazinyl, N-alkylpiperazinyl and azepinyl, or alternatively $R_2$ denotes a cycloalkyl radical containing 3 to 7 carbon atoms or a saturated 4-to 7-membered heterocyclic system chosen from azetidine, pyrrolidine, piperidine and azepine rings, these heterocyclic systems optionally being able to be substituted on the nitrogen atom with an alkyl radical, or $R_1$ denotes a formyl or alkylcarbonyl radical and $R_2$ denotes an alkyl radical substituted with a carboxy, alkylamino or dialkylamino radical in which the alkyl radicals optionally form, with the nitrogen atom to which they are attached, a 4- to 7-membered heterocyclic radical chosen from azetidinyl, pyrrolidinyl, piperidyl, piperazinyl, N-alkylpiperazinyl and azepinyl, or alternatively $R_2$ denotes a saturated 4- to 7-membered heterocyclic system chosen from azetidine, pyrrolidine, piperidine and azepine rings, these heterocyclic systems optionally being able to be substituted on

the nitrogen atom with an alkyl radical,

or $R_1$ and $R_2$, which may be identical or different, denote an alkyl radical optionally substituted with a carboxy, alkyloxycarbonyl, hydroxy, alkylamino or dialkylamino radical in which the alkyl radicals optionally form, with the nitrogen atom to which they are attached, a 4- to 7-membered heterocyclic radical chosen from azetidinyl, pyrrolidinyl, piperidyl, piperazinyl, N-alkylpiperazinyl and azepinyl,

or $R_1$ and $R_2$ form, together with the nitrogen atom to which they are linked, a 4- to 7-membered heterocyclic system chosen from azetidine, pyrrolidine, piperidine, morpholine and piperazine rings, optionally substituted with an alkyl radical, all the alkyl radicals and alkyl portions containing 1 to 5 carbon atoms in a straight or branched chain,

as well as the pharmaceutically acceptable salts thereof.

2. Process for preparing synergistin derivatives according to Claim 1 except for those for which $R_1$ denotes a formyl or alkylcarbonyl radical, characterized in that an amine of general formula:

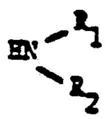

(II)

in which $R_1$ and $R_2$ are defined as in Claim 1 except for denoting a formyl or alkylcarbonyl radical, is reacted with a synergistin of general formula:

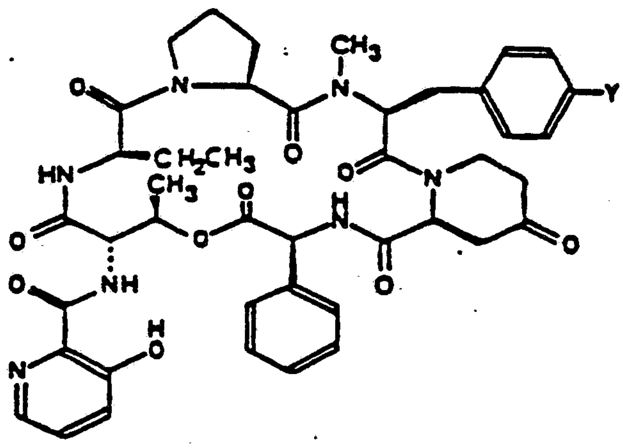

(III)

in which Y is defined as in Claim 1, in the presence of an alkali metal cyanoborohydride and then, where appropriate, the amine-protecting group is removed and the product obtained is isolated and optionally converted to a pharmaceutically acceptable salt.

3. Process for preparing synergistin derivatives according to Claim 1, in the formula of which derivatives $R_1$ denotes a formyl or alkylcarbonyl radical, $R_2$ denotes an alkyl radical substituted with a carboxy, alkylamino or dialkylamino radical in which the alkyl radicals form, together with the nitrogen atom to which they are attached a 4- to 7-membered heterocyclic radical as defined in Claim 1 or denotes a saturated 4- to 7-membered heterocyclic system chosen from azetidine, pyrrolidine, piperidine and azepine rings, these heterocyclic systems being able to be substituted on the nitrogen atom with an alkyl radical, and Y is defined as in Claim 1, characterized in that a product of general formula:

R - CO - X (IV)

in which R denotes a hydrogen atom or an alkyl radical and X denotes a halogen atom or an alkylcarbonyloxy radical, is reacted with a product of general formula:

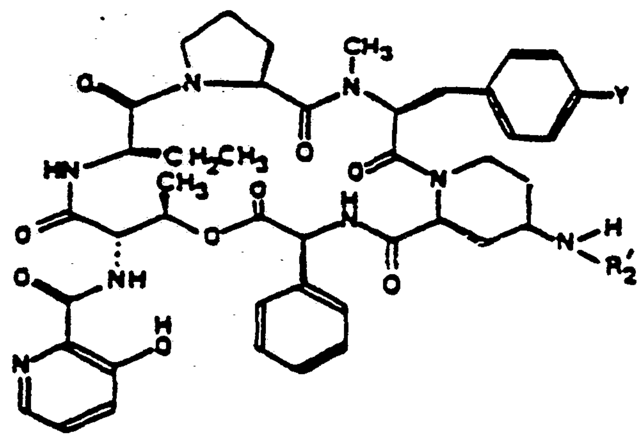

(V)

in which Y is defined as in Claim 1 and $R'_2$ has the corresponding definition of $R_2$ given above, and then, where appropriate, the amine-protecting group is removed and the product obtained is isolated and optionally converted to a pharmaceutically acceptable salt.

4. Process for preparing synergistin derivatives according to Claim 1, in the formula of which derivatives $R_1$ denotes a hydrogen atom, $R_2$ denotes a hydroxy radical and Y is defined as in Claim 1, characterized in that a product of general formula:

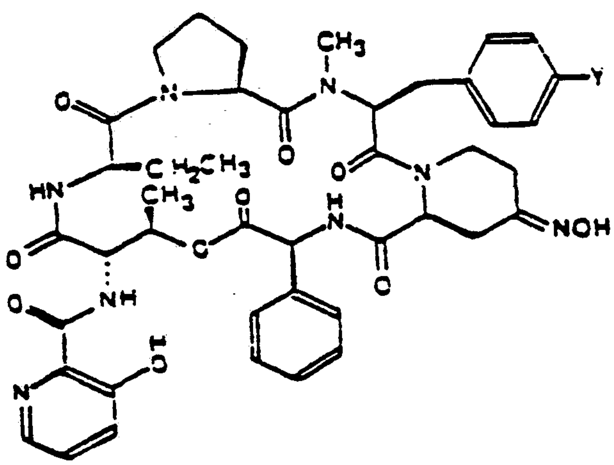

(VI)

in which Y is defined as in Claim 1, is reduced by any method known per se, and the product obtained is then isolated and optionally converted to a pharmaceutically acceptable salt.

5. Pharmaceutical composition, characterized in that it contains at least one derivative according to Claim 1, in combination with one or more diluents or adjuvants which are pharmaceutically compatible.

**Claim** for the contracting state AT

A process for preparing new synergistin derivatives of general formula:

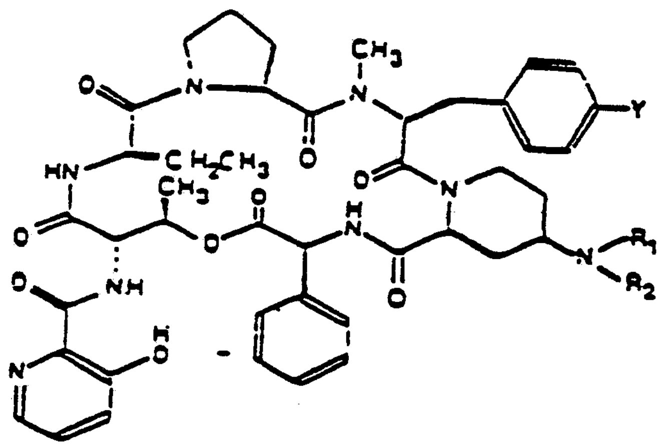

(I)

in which Y denotes a hydrogen atom or a dimethylamino radical and

either $R_1$ denotes a hydrogen atom and $R_2$ denotes a hydroxy radical or alkyl radical optionally substituted with a carboxy, alkyloxycarbonyl, hydroxy, alkylamino or dialkylamino radical in which the alkyl radicals optionally form, with the nitrogen atom to which they are attached, a 4-to 7-membered heterocyclic radical chosen from azetidinyl, pyrrolidinyl, piperidyl, piperazinyl, N-alkylpiperazinyl and azepinyl, or alternatively $R_2$ denotes a cycloalkyl radical containing 3 to 7 carbon atoms or a saturated 4-to 7-membered heterocyclic system chosen from azetidine, pyrrolidine, piperidine and azepine rings, these heterocyclic systems optionally being able to be substituted on the nitrogen atom with an alkyl radical,

or $R_1$ denotes a formyl or alkylcarbonyl radical and $R_2$ denotes an alkyl radical substituted with a carboxy, alkylamino or dialkylamino radical in which the alkyl radicals optionally form, with the nitrogen atom to which they are attached, a 4- to 7-membered heterocyclic radical chosen from azetidinyl, pyrrolidinyl, piperidyl, piperazinyl, N-alkylpiperazinyl and azepinyl, or alternatively $R_2$ denotes a saturated 4- to 7-membered heterocyclic system chosen from azetidine, pyrrolidine, piperidine and azepine rings, these heterocyclic systems optionally being able to be substituted on the nitrogen atom with an alkyl radical,

or $R_1$ and $R_2$, which may be identical or different, denote an alkyl radical optionally substituted with a carboxy, alkyloxycarbonyl, hydroxy, alkylamino or dialkylamino radical in which the alkyl radicals optionally form, with the nitrogen atom to which they are attached, a 4-to 7-membered heterocyclic radical chosen from azetidinyl, pyrrolidinyl, piperidyl, piperazinyl, N-alkylpiperazinyl and azepinyl,

or $R_1$ and $R_2$ form, together with the nitrogen atom to which they are linked, a 4- to 7-membered heterocyclic system chosen from azetidine, pyrrolidine, piperidine, morpholine and piperazine rings, optionally substituted with an alkyl radical, all the alkyl radicals and alkyl portions containing 1 to 5 carbon atoms in a straight or branched chain,

as well as the pharmaceutitally acceptable salts thereof, characterized in that

when $R_1$ is other than a formyl or alkylcarbonyl radical, and $R_2$ is defined as above, an amine of general formula:

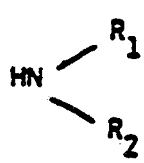

(II)

in which $R_1$ and $R_2$ are defined as above, is reacted with a synergistin of general formula:

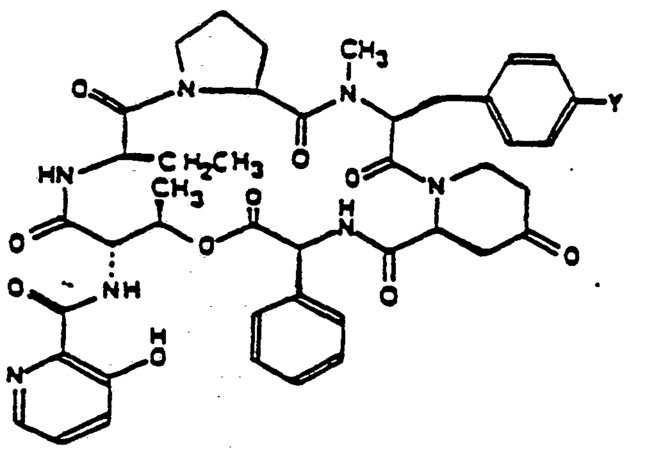

(III)

in which Y is defined as above, in the presence of an alkali metal cyanoborohydride, and then, where appropriate, the amine-protecting group is removed, or alternatively, when $R_1$ denotes a formyl or alkylcarbonyl radical, $R_2$ denotes an alkyl radical substituted with a carboxy, alkylamino or dialkylamino radical in which the alkyl radicals form, together with the nitrogen atom to which they are attached, a 4- to 7-membered heterocyclic radical as defined above or denotes a saturated 4- to 7-membered heterocyclic system chosen from azetidine, pyrrolidine, piperidine and azepine rings, these heterocyclic systems being able to be substituted on the nitrogen atom with an alkyl radical, and Y is defined as above, a product of general formula:

R - CO - X (IV)

in which R denotes a hydrogen atom or an alkyl radical and X denotes a halogen atom or an alkylcarbonyloxy radical, is reacted with a product of general formula:

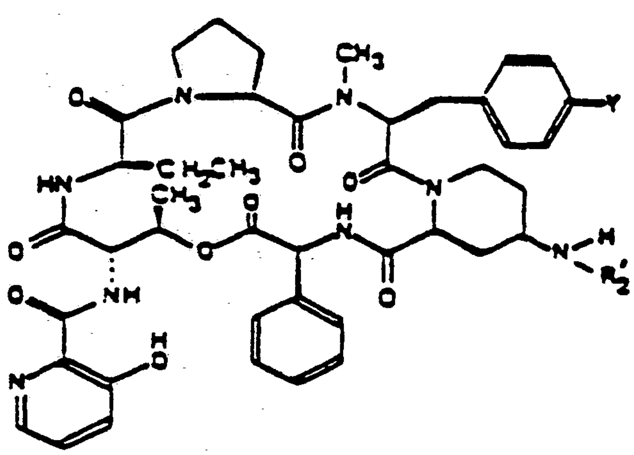

(V)

in which Y is defined as above and $R'_2$ has the corresponding definition of $R_2$ given above, and then, where appropriate, the amine-protecting group is removed, or alternatively, when $R_1$ denotes a hydrogen atom, $R_2$ denotes a hydroxy radical and Y is defined as above, a product of general formula:

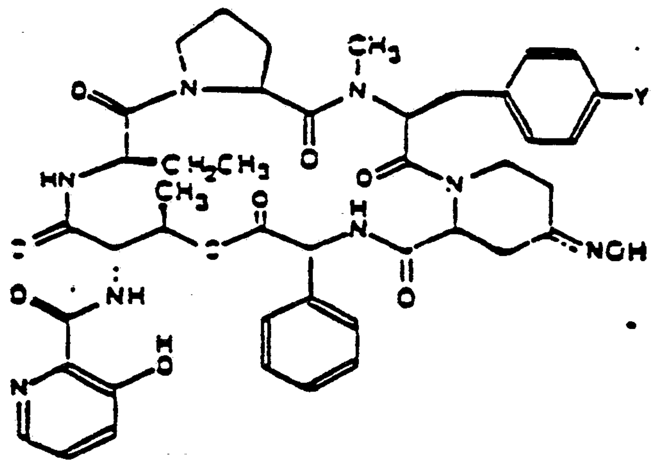

(VI)

in which Y is defined as above, is reduced by any method known per se and the product obtained is then isolated and optionally converted to a pharmaceutically acceptable salt.